# EUROPEAN PATENT APPLICATION

(11) **EP 1 312 363 A1**
(43) Date of publication of application: **21.05.2003**
(21) Application number: 02256371.2
(22) Date of filing: 13.09.2002
(51) Int. Cl.: A61K 31/495, A61K 31/437, A61P 25/00, A61P 1/10

(54) **Methods of treatment and kits comprising a growth hormone secretagogue**

(30) Priority: 28.09.2001 US 325921 P
(71) Applicant: Pfizer Products Inc., Groton, Connecticut 06340 (US)
(72) Inventor: Carpino, Philip Albert, Groton, Connecticut 06340 (US); Griffith, David Andrew, Groton, Connecticut 06340 (US); Hadcock, John Richard Neville, Groton, Connecticut 06340 (US); Landschulz, William Harras, Groton, Connecticut 06340 (US); Pan, Lydia Codetta, Groton, Connecticut 06340 (US)
(74) Representative: Sewell, Richard Charles

(57) **Abstract**

The present invention relates to methods of treating bulimia nervosa, male erectile dysfunction, female sexual dysfunction, thyroid cancer, breast cancer, or ameliorating ischemic nerve or muscle damage. The present invention also relates to kits that can be used in the treatment of bulimia nervosa, male erectile dysfunction, female sexual dysfunction, thyroid cancer, breast cancer, or ameliorating ischemic nerve or muscle damage. The present invention further relates to increasing gastrointestinal motility after surgery and increasing gastrointestinal motility in patients who have been administered an agent that decreases gastrointestinal motility.

## Description

### Field of the Invention

The present invention relates to methods of treating bulimia nervosa, male erectile dysfunction, female sexual dysfunction, thyroid cancer, breast cancer, or ameliorating ischemic nerve or muscle damage. The present invention also relates to kits that can be used in the treatment of bulimia nervosa, male erectile dysfunction, female sexual dysfunction, thyroid cancer, breast cancer, or ameliorating ischemic nerve or muscle damage. The present invention also relates to increasing gastrointestinal motility after surgery and increasing gastrointestinal motility in patients who have been administered an agent that decreases gastrointestinal motility.

### Background of the Invention

The present invention relates to new treatments and kits comprising a growth hormone secretagogue.

Growth hormone, which is secreted from the pituitary gland, stimulates growth of all tissues of the body that are capable of growing. In addition, growth hormone is known to have the following basic effects on the metabolic processes of the body: (1) increased rate of protein synthesis in all cells of the body; (2) decreased rate of carbohydrate utilization in cells of the body; and (3) increased mobilization of free fatty acids and use of fatty acids for energy. As is known to those skilled in the art, the known and potential uses of growth hormone are varied and multitudinous. See "Human Growth Hormone," Strobel and Thomas, Pharmacological Reviews, 46, pg. 1-34 (1994). Also, these varied uses of growth hormone are summarized in International Patent Application Publication Number WO 97/24369.

Various ways are known to release growth hormone (see Recent Progress in Hormone Research, vol. 52, pp. 215-245 (1997); and Front. Horm. Res. Basel, Karger, vol. 24, pp. 152-175 (1999)). For example, chemicals such as arginine, L-3,4-dihydroxyphenylalanine (L-DOPA), glucagon, vasopressin, and insulin induced hypoglycemia, as well as activities such as sleep and exercise, indirectly cause growth hormone to be released from the pituitary by acting in some fashion on the hypothalamus, perhaps either to decrease somatostatin secretion or to increase secretion of growth hormone releasing factor (GRF) or ghrelin (see Nature, vol. 402, pp. 656-660 (9 December 1999)), or all of these. Moreover, it has recently been discovered that growth hormone secretagogues act as ghrelin mimetics. Ghrelin is a protein that binds to the growth hormone secretagogue receptor.

In cases where increased levels of growth hormone were desired, the problem was generally solved by providing exogenous growth hormone or by administering GRF, IGF-I or a peptidyl compound that stimulated growth hormone production and/or release. In any case, the peptidyl nature of the compound necessitated that it be administered by injection. Initially, the source of growth hormone was the extraction of the pituitary glands of cadavers. This resulted in a very expensive product and carried with it the risk that a disease associated with the source of the pituitary gland could be transmitted to the recipient of the growth hormone. Recombinant growth hormone has become available which, while no longer carrying any risk of disease transmission, is still a very expensive product which must be given by injection. In addition, administration of exogenous growth hormone may result in side-effects, including edema, and does not correlate with the pulsatile release seen in the endogenous release of growth hormone.

Certain compounds have been developed which stimulate the release of endogenous growth hormone. Peptides which are known to stimulate the release of endogenous growth hormone include growth hormone releasing hormone and its analogs, the growth hormone releasing peptides, GHRP-6 and GHRP-1 (described in U.S. Patent No. 4,411,890; International Patent Application Publication No. WO 89/07110; and International Patent Application Publication No. WO 89/07111), and GHRP-2 (described in International Patent Application Publication No. WO 93/04081), as well as hexarelin (J. Endocrinol. Invest., 15 (Suppl. 4): 45 (1992)).

Other compounds possessing growth hormone secretagogue activity are disclosed in the following International Patent Applications (listed by Publication Nos.), issued U.S. Patents and published European Patent Applications: WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO 97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

In addition, the following growth hormone secretagogues are known in the art: MK-0677, L-162752 and L-163022 (Merck); NN703 and ipamorelin (Novo Nordisk); hexarelin (Pharmacia & Upjohn); GPA-748 (KP102, GHRP-2) (American Home Products); and LY444711 (Eli Lilly). The following agents that stimulate GH release via GHRH/GRF receptor (including GHRH/GRF derivatives, analogs and mimetics) are known in the art: Geref (Ares/Serono); GHRH (1-44) (BioNebraska); Somatorelin (GRF 1-44) (Fujisawa/ICN); and ThGRF (Theratechnologies).

Endocrine Reviews 18(5): 621-645 (1997) provides an overview of peptidomimetic regulation of growth hormone secretion by growth hormone secretagogues. Horm. Res. 1999; 51(suppl 3):16-20 (1999), examines the clinical and experimental effects of growth hormone secretagogues on various organ systems. Drug Discovery Today, Vol. 4, No. 11, November 1999; and TEM Vol. 10, No. 1, 1999, disclose potential therapeutic applications of growth hormone secretagogues, including their use in treating growth hormone disorders such as growth hormone deficiency (GHD), age-related conditions, obesity and catabolic conditions, and their use in sleep enhancement.

International Patent Application Publication Nos. WO 97/24369 and WO 98/58947 disclose that certain growth hormone secretagogues are useful for the treatment or prevention of osteoporosis, congestive heart failure, frailty associated with aging, obesity, accelerating bone fracture repair, attenuating protein catabolic response after a major operation, reducing cachexia and protein loss due to chronic illness, accelerating wound healing or accelerating the recovery of burn patients or patients having undergone major surgery, improving muscle strength, mobility, maintenance of skin thickness, metabolic homeostasis or renal homeostasis. Published European patent application 0995748 discloses that certain dipeptide growth hormone secretagogues are useful for the treatment or prevention of musculoskeletal frailty, including osteoporosis.

### Summary of the Invention

The present invention provides methods of treating bulimia nervosa, male erectile dysfunction, female sexual dysfunction, thyroid cancer, or breast cancer, or ameliorating ischemic nerve or muscle damage, the methods comprising administering to a patient in need thereof a therapeutically effective amount of a compound that is a growth hormone secretagogue or a pharmaceutically acceptable salt of prodrug thereof.

In a preferred embodiment of the methods, the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide or 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide or a pharmaceutically acceptable salt or prodrug thereof.

In another preferred embodiment of the methods, the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide, L-tartrate or the L-(+)-tartaric acid salt of 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide.

Also provided are methods of increasing gastrointestinal motility comprising administering to a patient who has taken or who is to take an agent that is known to decrease gastrointestinal motility a therapeutically effective amount of a growth hormone secretagogue or a pharmaceutically acceptable salt or prodrug thereof.

In a preferred embodiment of the methods of increasing gastrointestinal motility, the agent that is known to decrease gastrointestinal motility is a calcium channel blocker, a beta blocker, or a narcotic.

In another preferred embodiment of the methods of increasing gastrointestinal motility, the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide or 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide or a pharmaceutically acceptable salt or prodrug thereof.

In another preferred embodiment of the methods of increasing gastrointestinal motility, the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide, L-tartrate or the L-(+)-tartaric acid salt of 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide.

Also provided are methods of increasing gastrointestinal motility after surgery, the methods comprising administering to a patient in need thereof a therapeutically effective amount of a growth hormone secretagogue or a pharmaceutically acceptable salt or prodrug thereof.

In a preferred embodiment of the methods of increasing gastrointestinal motility after surgery, the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide or 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide or a pharmaceutically acceptable salt or prodrug thereof.

In another preferred embodiment of the methods of increasing gastrointestinal motility after surgery, the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide, L-tartrate or the L-(+)-tartaric acid salt of 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide.

Also provided are kits for the treatment of bulimia nervosa, male erectile dysfunction, female sexual dysfunction, thyroid cancer, or breast cancer, or ameliorating ischemic nerve or muscle damage, the kits comprising:
a. a pharmaceutical composition that comprises a growth hormone secretagogue or a pharmaceutically acceptable salt or prodrug thereof; and
b. instructions for administering the pharmaceutical composition to a patient in need thereof to treat bulimia nervosa, male erectile dysfunction, female sexual dysfunction, thyroid cancer, or breast cancer, or ameliorating ischemic nerve or muscle damage.

Also provided are kits for the treatment of bulimia nervosa, male erectile dysfunction, female sexual dysfunction, thyroid cancer, or breast cancer, or ameliorating ischemic nerve or muscle damage, the kits comprising:
a. a first pharmaceutical composition that comprises a growth hormone secretagogue or a pharmaceutically acceptable salt or prodrug thereof;
b. a second pharmaceutical composition that comprises a second compound that can be used to treat bulimia nervosa, male erectile dysfunction, female sexual dysfunction, thyroid cancer, or breast cancer, or ameliorating ischemic nerve or muscle damage; and
c. instructions for administering the pharmaceutical compositions to a patient in need thereof to treat bulimia nervosa, male erectile dysfunction, female sexual dysfunction, thyroid cancer, or breast cancer, or ameliorating ischemic nerve or muscle damage.

### Detailed Description of the Invention

A growth hormone secretagogue can be used to treat bulimia nervosa, male erectile dysfunction, female sexual dysfunction, thyroid cancer, breast cancer, or ameliorate ischemic nerve or muscle damage. A growth hormone secretagogue can also be used to increase gastrointestinal motility after surgery and increase gastrointestinal motility in patients who have been administered or who are to be administered an agent that decreases gastrointestinal motility.

The disease bulimia nervosa is characterized by episodes of binge eating where a large quantity of food is consumed followed by use of laxatives, self-induced vomiting, use of diuretics, vigorous exercise, or fasting to avoid weight gain. Bulimia primarily affects young women, but can also affect men. Bulimia is typically treated using psychotherapy and/or antidepressants.

Ischemic nerve and muscle damage is damage that occurs to nerve and muscle tissue as a result of ischemia. Ischemia is reduced blood flow to a tissue. Ischemic damage of muscle tissue includes skeletal muscle and cardiac muscle. Ischemia can occur in cardiac muscle as a result of a myocardial infarction, hemorrhage or other cardiac events.

Male erectile dysfunction is the inability of a male to attain or sustain an erection satisfactory for coitus.

Female sexual dysfunction (FSD) has several components including sexual arousal disorder, decreased libido, hypoactive sexual desire disorder, sexual ahedonia, and dyspareunia. Each of these components can be considered separate conditions and treated separately with a growth hormone secretagogue. A patient suffering from FSD may have symptoms of more than one component.

Proper sexual functioning in women depends on the sexual response cycle, which consists of an anticipatory mental set (sexual motive state or state of desire), effective vasocongestive arousal (swelling and lubrication), orgasm, and resolution. In women, orgasm is accompanied by contractions (not always subjectively experienced as such) of the muscles of the outer third of the vagina. Generalized muscular tension, perineal contractions, and involuntary pelvic thrusting (every 0.8 sec) usually occur. Orgasm is followed by resolution--a sense of general pleasure, well-being, and muscular relaxation. During this phase, women may be able to respond to additional stimulation almost immediately.

The sexual response cycle is mediated by a delicate, balanced interplay between the sympathetic and parasympathetic nervous systems. Vasocongestion is largely mediated by parasympathetic (cholinergic) outflow; orgasm is predominantly sympathetic (adrenergic). These responses are easily inhibited by cortical influences or by impaired hormonal, neural, or vascular mechanisms. Disorders of sexual response may involve one or more of the cycle's phases. Generally, both the subjective components of desire, arousal, and pleasure and the objective components of performance, vasocongestion, and orgasm are disturbed, although any may be affected independently. Sexual dysfunctions may be lifelong (no effective performance ever, generally due to intrapsychic conflicts) or acquired (after a period of normal function); generalized or limited to certain situations or certain partners; and total or partial.

Hypoactive sexual desire disorder is a disorder in which sexual fantasies and desire for sexual activity are persistently or recurrently diminished or absent, causing marked distress or interpersonal difficulties. Hypoactive sexual desire disorder may be lifelong or acquired, generalized (global) or situational (partner-specific). Sexual desire is a complex psychosomatic process based on brain activity (the "generator" or "motor" running in a rheostatic cyclic fashion), a poorly defined hormonal milieu, and cognitive scripting that includes sexual aspiration and motivation. Desynchronization of these components results in hypoactive sexual desire disorder.

The acquired form of hypoactive sexual desire disorder is commonly caused by boredom or unhappiness in a long-standing relationship, depression (which leads more often to decreased interest in sex than it does to impotence in the male or to inhibited excitement in the female), dependence on alcohol or psychoactive drugs, side effects from prescription drugs (e.g., antihypertensives, antidepressants), and hormonal deficiencies. This disorder can be secondary to impaired sexual functioning in the arousal or orgasm phase of the sexual response cycle.

Symptoms and signs of hypoactive sexual desire disorder include the patient complaining of a lack of interest in sex, even in ordinarily erotic situations. The disorder is usually associated with infrequent sexual activity, often causing serious marital conflict. Some patients have sexual encounters fairly often to please their partners and may have no difficulty with performance but continue to have sexual apathy. When boredom is the cause, frequency of sex with the usual partner decreases, but sexual desire may be normal or even intense with others (the situational form).

Clinically significant sexual dysfunction that causes personal distress or interpersonal problems and is most likely fully explained by direct physiologic effects of a physical disorder. Sexual dysfunction due to a physical disorder is usually generalized (not specific to a given partner or situation). It is diagnosed when evidence from a patient's history, physical examination, or laboratory assessment can explain the dysfunction physiologically and when mental disorders that may better explain it can be ruled out. Resolution of the underlying physical disorders often results in resolution or amelioration of the sexual dysfunction. When the cause of sexual dysfunction is a combination of psychologic and physical factors, the appropriate diagnosis is sexual dysfunction due to combined factors.

Sexual anhedonia (decreased or absent pleasure in sexual activity) is not an official diagnosis. It is almost always classified under hypoactive sexual desire disorder, because loss of pleasure almost always results in loss of desire (although loss of desire may occur first). The cause is likely to be depression or drugs if anhedonia is acquired and global (with all partners in all situations); interpersonal factors if anhedonia is confined to one partner or one situation; or repressive factors (e.g., guilt, shame) due to family dysfunction or childhood trauma if anhedonia is lifelong. Sexual aversion is the probable diagnosis in lifelong cases.

Sexual arousal disorder is the persistent or recurrent inability to attain or to maintain the lubrication-swelling response of sexual excitement until completion of sexual activity. This disturbance occurs despite adequate focus, intensity, and duration of sexual stimulation. The disorder may be lifelong or, more commonly, acquired and restricted to the partner. The patient's complaints are usually related to lack of orgasm, although some women report lack of excitement.

Although women can be orgasmic throughout their lives, sexual activity often decreases after age 60 because of the relative lack of partners and untreated physiologic changes (e.g., atrophy of the vaginal mucosa, with resultant dryness and painful coitus).

The female sexual response phase of arousal is not easily distinguished from the phase of desire until physiological changes begin to take place in the vagina and clitoris as well as other sexual organs. Sexual excitement and pleasure are accompanied by a combination of vascular and neuromuscular events which lead to engorgement of the clitoris, labia and vaginal wall, increased vaginal lubrication and dilatation of the vaginal lumen (Levin, R.J., Clin. Obstet. Gynecol., 1980:7; 213-252; Ottesen, B., Gerstenberg, T., Ulrichsen, H. et al., Eur. J. Clin. Invest., 1983:13; 321-324; Levin, R.J., Exp. Clin. Endocrinol., 1991:98; 61-69; Levin, R.J., Ann. Rev. Sex Res., 1992:3; 1-48; Masters, W. H., Johnson, V. E. Human Sexual Response. Little, Brown: Boston, 1996; Berman, J.R., Berman, L. & Goldstein, L., Urology, 1999:54; 385-391).

Vaginal engorgement enables transudation to occur and this process is responsible for increased vaginal lubrication. Transudation allows a flow of plasma through the epithelium and onto the vaginal surface, the driving force for which is increased blood flow in the vaginal capillary bed during the aroused state. In addition engorgement leads to an increase in vaginal length and luminal diameter, especially in the distal two-thirds of the vaginal canal. The luminal dilatation of the vagina is due to a combination of smooth muscle relaxation of its wall and skeletal muscle relaxation of the pelvic floor muscles. Some sexual pain disorders such as vaginismus are thought to be due, at least in part, by inadequate relaxation preventing dilatation of the vagina; it has yet to be ascertained if this is primarily a smooth or skeletal muscle problem. (Masters, W. H., Johnson, V. E. Human Sexual Response. Little, Brown: Boston, 1996; Berman, J.R., Berman, L. & Goldstein, L., Urology, 1999:54; 385-391).

The components of FSD are best defined by contrasting them to the phases of normal female sexual response: desire, arousal and orgasm. Desire or libido is the drive for sexual expression. Its manifestations often include sexual thoughts either when in the company of an interested partner or when exposed to other erotic stimuli. Arousal is the vascular response to sexual stimulation, an important component of which is vaginal lubrication and elongation of the vagina. Orgasm is the release of sexual tension that has culminated during arousal.

Hence, FSD occurs when a woman has an inadequate or unsatisfactory response in any of these phases; desire, arousal or orgasm. FSD categories include hypoactive sexual desire disorder, sexual arousal disorder, orgasmic disorders and sexual pain disorders.

Hypoactive sexual desire disorder is present if a woman has no or little desire to be sexual, and has no or few sexual thoughts or fantasies. This component of FSD can be caused by low testosterone levels, due either to natural menopause or to surgical menopause. Other causes include illness, medications, fatigue, depression and anxiety.

Sexual arousal disorder (FSAD) is characterized by inadequate genital response to sexual stimulation. The genitalia do not undergo the engorgement that characterizes normal sexual arousal. The vaginal walls are poorly lubricated, so that intercourse is painful. Orgasms may be impeded. Arousal disorder can be caused by reduced estrogen at menopause or after childbirth and during lactation, as well as by illnesses, with vascular components such as diabetes and atherosclerosis. Other causes result from treatment with diuretics, antihistamines, antidepressants, e.g., SSRIs or antihypertensive agents.

Sexual pain disorders (including dyspareunia and vaginismus) are characterized by pain resulting from penetration and may be caused by medications which reduce lubrication, endometriosis, pelvic inflammatory disease, inflammatory bowel disease or urinary tract problems.

Dyspareunia is painful coitus or attempted coitus. Dyspareunia can occur before, during, or after intercourse. Causes include menopausal involution with dryness and thinning of the mucosa. Pain during or after coitus is the chief complaint.

Examples of growth hormone secretagogues that can be used in the present methods and kits include, but are not limited, to a compound of the Formula I: 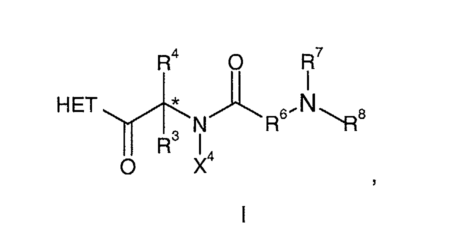 or a stereoisomeric mixture thereof, diastereomerically enriched, diastereomerically pure, enantiomerically enriched or enantiomerically pure isomer thereof, or a prodrug of such compound, mixture or isomer thereof, or a pharmaceutically acceptable salt of the compound, mixture, isomer or prodrug, or a tautomer thereof, wherein
HET is a heterocyclic moiety selected from the group consisting of 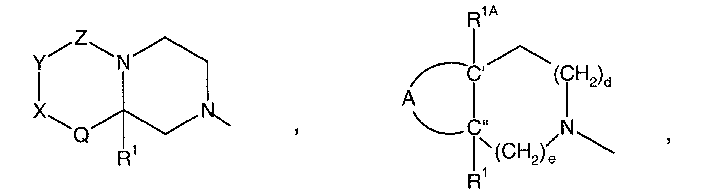 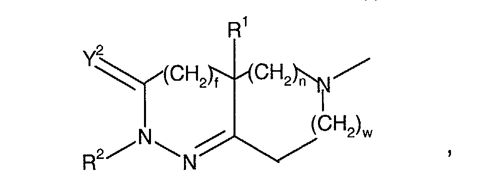 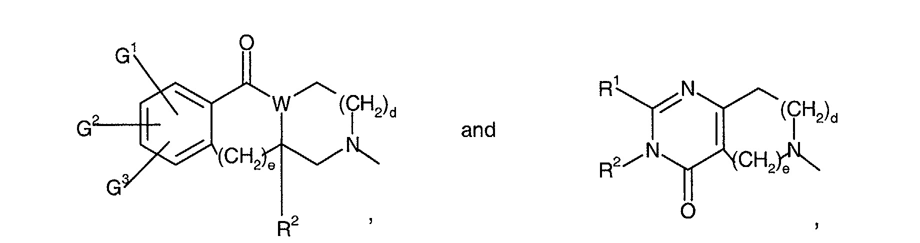 d is 0, 1 or 2;
e is 1 or 2;
f is 0 or 1;
n and w are 0, 1 or 2, provided that n and w cannot both be 0 at the same time;
Y² is oxygen or sulfur;
A is a divalent radical, where the left hand side of the radical as shown below is connected to C" and the right hand side of the radical as shown below is connected to C', selected from the group consisting of
-NR²-C(O)-NR²-, -NR²-S(O)₂-NR²-, -O-C(O)-NR²-, -NR²-C(O)-O-, -C(O)-NR²-C(O)-, -C(O)-NR²-C(R⁹R¹⁰)-, -C(R⁹R¹⁰)-NR²-C(O)-, -C(R⁹R¹⁰)-C(R⁹R¹⁰)-C(R⁹R¹⁰)-, -S(O)₂-C(R⁹R¹⁰)-C(R⁹R¹⁰)-, -C(R⁹R¹⁰)-O-C(O)-, -C(R⁹R¹⁰)-O-C(R⁹R¹⁰)-, -NR²-C(O)-C(R⁹R¹⁰)-, -O-C(O)-C(R⁹R¹⁰)-, -C(R⁹R¹⁰)-C(O)-NR²-, -C(R⁹R¹⁰)-C(O)-O-, -C(O)-NR²-C(R⁹R¹⁰)-C(R⁹R¹⁰)-, -C(O)-O-C(R⁹R¹⁰)-, -C(R⁹R¹⁰)-C(R⁹R¹⁰)-C(R⁹R¹⁰)-C(R⁹R¹⁰)-,-S(O)₂-NR²-C(R⁹R¹⁰)-C(R⁹R¹⁰)-, -C(R⁹R¹⁰)-C(R⁹R¹⁰)-NR²-C(O)-, -C(R⁹R¹⁰)-C(R⁹R¹⁰)-O-C(O)-, -NR²-C(O)-C(R⁹R¹⁰)-C(R⁹R¹⁰)-, -NR²-S(O)₂-C(R⁹R¹⁰)-C(R⁹R¹⁰)-, -O-C(O)-C(R⁹R¹⁰)-C(R⁹R¹⁰)-, -C(R⁹R¹⁰)-C(R⁹R¹⁰)-C(O)-NR²-, -C(R⁹R¹⁰)-C(R⁹R¹⁰)-C(O)-,-C(R⁹R¹⁰)-NR²-C(O)-O-, -C(R⁹R¹⁰)-O-C(O)-NR², -C(R⁹R¹⁰)-NR²-C(O)-NR²-, -NR²-C(O)-O-C(R⁹R¹⁰)-, -NR²-C(O)-NR²-C(R⁹R¹⁰)-, -NR²-S(O)₂-NR²-C(R⁹R¹⁰)-, -O-C(O)-NR²-C(R⁹R¹⁰)-, -C(O)-N=C(R¹¹)-NR²-, -C(O)-NR²-C(R¹¹)=N-, -C(R⁹R¹⁰)-NR¹²-C(R⁹R¹⁰)-, -NR¹²-C(R⁹R¹⁰)-, -NR¹²-C(R⁹R¹⁰)-C(R⁹R¹⁰)-, -C(O)-O-C(R⁹R¹⁰)-C(R⁹R¹⁰)-, -NR²-C(R¹¹)=N-C(O)-, -C(R⁹R¹⁰)-C(R⁹R¹⁰)-N(R¹²)-, -C(R⁹R¹⁰)-NR¹²-, -N=C(R¹¹)-NR²-C(O)-, -C(R⁹R¹⁰)-C(R⁹R¹⁰)-NR²-S(O)₂-, -C(R⁹R¹⁰)-C(R⁹R¹⁰)-S(O)₂-NR²-, -C(R⁹R¹⁰)-C(R⁹R¹⁰)-C(O)-O-, -C(R⁹R¹⁰)-S(O)₂-C(R⁹R¹⁰)-, -C(R⁹R¹⁰)-C(R⁹R¹⁰)-S(O)₂-, -O-C(R⁹R¹⁰)-C(R⁹R¹⁰)-, -C(R⁹R¹⁰)-C(R⁹R¹⁰)-O-, -C(R⁹R¹⁰)-C(O)-C(R⁹R¹⁰)-, -C(O)-C(R⁹R¹⁰)-C(R⁹R¹⁰)- and -C(R⁹R¹⁰)-NR²-S(O)₂-NR²-;
Q is a covalent bond or CH₂;
W is CH or N;
X is CR⁹R¹⁰, C=CH₂ or C=O;
Y is CR⁹R¹⁰, O or NR²;
Z is C=O, C=S or S(O)₂;
G¹ is hydrogen, halo, hydroxy, nitro, amino, cyano, phenyl, carboxyl, -CONH₂, -(C₁-C₄)alkyl optionally independently substituted with one or more phenyl, one or more halogens or one or more hydroxy groups, -(C₁-C₄)alkoxy optionally independently substituted with one or more phenyl, one or more halogens or one or more hydroxy groups, -(C₁-C₄)alkylthio, phenoxy, -COO(C₁-C₄)alkyl, N,N-di-(C₁-C₄)alkylamino, -(C₂-C₆)alkenyl optionally independently substituted with one or more phenyl, one or more halogens or one or more hydroxy groups, -(C₂-C₆)alkynyl optionally independently substituted with one or more phenyl, one or more halogens or one or more hydroxy groups, -(C₃-C₆)cycloalkyl optionally independently substituted with one or more (C₁-C₄)alkyl groups, one or more halogens or one or more hydroxy groups, -(C₁-C₄)alkylamino carbonyl or di-(C₁-C₄)alkylamino carbonyl; G² and G³ are each independently selected from the group consisting of hydrogen, halo, hydroxy, -(C₁-C₄)alkyl optionally independently substituted with one to three halo groups and -(C₁-C₄)alkoxy optionally independently substituted with one to three halo groups;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)S(O)₂(CH₂)ₜ-A¹, -(CH₂)_{q}N(X⁶)S(O)₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)S(O)₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro groups;
Y¹ is O, S(O)ₘ, -C(O)NX⁶-, -CH=CH-, -C≡C-, -N(X⁶)C(O)-, -C(O)NX⁶-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group in the definition of R¹ are optionally independently substituted with hydroxy, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro groups or 1 or 2 (C₁-C₄)alkyl groups;
R^{1A} is selected from the group consisting of hydrogen, F, Cl, Br, I, (C₁-C₆)alkyl, phenyl(C₁-C₃)alkyl, pyridyl(C₁-C₃)alkyl, thiazolyl(C₁-C₃)alkyl and thienyl(C₁-C₃)alkyl, provided that R^{1A} is not F, Cl, Br or I when a heteroatom is vicinal to C";
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxy, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 independently selected halo groups;
R³ is selected from the group consisting of A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ and -(C₁-C₅)alkyl-X¹-(C₁-C₅)alkyl-(C₃-C₇)cycloalkyl;
where the alkyl groups in the definition of R³ are optionally substituted with -S(O)ₘ(C₁-C₆)alkyl, -C(O)OX³, 1, 2, 3, 4 or 5 independently selected halo groups or 1, 2 or 3 independently selected -OX³ groups;
X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl, or R⁴ is taken together with R³ and the carbon atom to which they are attached and form (C₅-C₇)cycloalkyl, (C₅-C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 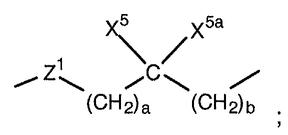 where a and b are each independently 0, 1, 2 or 3;
X⁵ and X^{5a} are each independently selected from the group consisting of hydrogen, CF₃, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
or the carbon bearing X⁵ or X^{5a} forms one or two alkylene bridges with the nitrogen atom bearing R⁷ and R⁸ wherein each alkylene bridge contains 1 to 5 carbon atoms, provided that when one alkylene bridge is formed then only one of X⁵ or X^{5a} is on the carbon atom and only one of R⁷ or R⁸ is on the nitrogen atom and further provided that when two alkylene bridges are formed then X⁵ and X^{5a} cannot be on the carbon atom and R⁷ and R⁸ cannot be on the nitrogen atom;
or X⁵ is taken together with X^{5a} and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen;
or X⁵ is taken together with X^{5a} and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
Z¹ is a bond, O or N-X², provided that when a and b are both 0 then Z¹ is not N-X² or O;
or R⁶ is -(CR^{a}R^{b})ₐ-E-(CR^{a}R^{b})_{b}-, where the -(CR^{a}R^{b})ₐ- group is attached to the carbonyl carbon of the amide group of the compound of formula I and the -(CR^{a}R^{b})_{b} group is attached to the terminal nitrogen atom of the compound of Formula I;
E is -O-, -S-, -CH=CH- or an aromatic moiety selected from 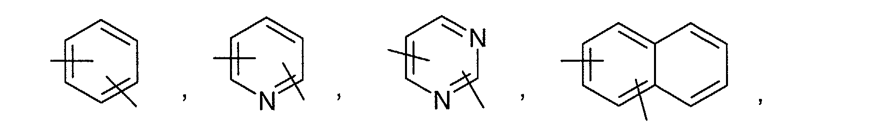 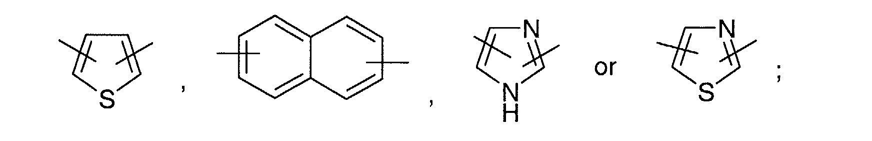 said aromatic moiety in the definition of E optionally substituted with up to three halo, hydroxy, -N(R^{c})(R^{c}), (C₁-C₆)alkyl or (C₁-C₆)alkoxy;
R^{a} and R^{b} are, for each occurrence, independently hydrogen, (C₁-C₆)alkyl, trifluoromethyl, phenyl or monosubstituted (C₁-C₆)alkyl where the substituents are imidazolyl, naphthyl, phenyl, indolyl, p-hydroxyphenyl, -OR^{c}, S(O)ₘR^{c}, C(O)OR^{c}, (C₃-C₇)cycloalkyl, -N(R^{c})(R^{c}), -C(O)N(R^{c})(R^{c}), or
R^{a} or R^{b} may independently be joined to one or both of R⁷ or E (where E is other than O, S or -CH=CH-) to form an alkylene bridge between the terminal nitrogen and the alkyl portion of the R^{a} or R^{b} and the R⁷ or E group, wherein the bridge contains 1 to 8 carbon atoms; or R^{a} and R^{b} may be joined to one another to form a (C₃-C₇)cycloalkyl;
R^{c}, for each occurrence, is independently hydrogen or (C₁-C₆)alkyl;
a and b are independently 0, 1, 2 or 3, with the proviso that if E is -O- or -S-, b is other than 0 or 1 and with the further proviso that if E is -CH=CH-, b is other than 0;
R⁷ and R⁸ are each independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halo groups, 1 to 3 hydroxy groups, 1 to 3 -O-C(O)(C₁-C₁₀)alkyl groups or 1 to 3 (C₁-C₆)alkoxy groups; or
R⁷ and R⁸ can be taken together to form -(CH₂)ᵣ-L-(CH₂)ᵣ-;
where L is C(X²)(X²), S(O)ₘ or N(X²);
R⁹ and R¹⁰ are each independently selected from the group consisting of hydrogen, fluoro, hydroxy and (C₁-C₅)alkyl optionally independently substituted with 1-5 halo groups;
R¹¹ is selected from the group consisting of (C₁-C₅)alkyl and phenyl optionally substituted with 1-3 substitutents each independently selected from the group consisting of (C₁-C₅)alkyl, halo and (C₁-C₅)alkoxy;
R¹² is selected from the group consisting of (C₁-C₅)alkylsulfonyl, (C₁-C₅)alkanoyl and (C₁-C₅)alkyl where the alkyl portion is optionally independently substituted by 1-5 halo groups;
A¹ for each occurrence is independently selected from the group consisting of (C₅-C₇)cycloalkenyl, phenyl, a partially saturated, fully saturated or fully unsaturated 4- to 8-membered ring optionally having 1 to 4 heteroatoms independently selected from the group consisting of oxygen, sulfur and nitrogen and a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ for each occurrence is independently optionally substituted, on one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1H-tetrazol-5-yl, phenyl, phenoxy, phenylalkyloxy, halophenyl, methylenedioxy, -N(X⁶)(X⁶), -N(X⁶)C(O)(X⁶), -S(O)₂N(X⁶)(X⁶), -N(X⁶)S(O)₂-phenyl, -N(X⁶)S(O)₂X⁶, -CONX¹¹X¹², -S(O)₂NX¹¹X¹², -NX⁶S(O)₂X¹², -NX⁶CONX¹¹X¹², -NX⁶S(O)₂NX¹¹X¹², -NX⁶C(O)X¹², imidazolyl, thiazolyl and tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl, 1 to 5 halo groups, 1 to 3 hydroxy groups, 1 to 3 (C₁-C₁₀)alkanoyloxy groups or 1 to 3 (C₁-C₆)alkoxy groups;
X¹² is hydrogen, (C₁-C₆)alkyl, phenyl, thiazolyl, imidazolyl, furyl or thienyl, provided that when X¹² is not hydrogen, the X¹² group is optionally substituted with one to three substituents independently selected from the group consisting of Cl, F, CH₃, OCH₃, OCF₃ and CF₃;
or X¹¹ and X¹² are taken together to form -(CH₂)ᵣ-L¹-(CH₂)ᵣ-;
L¹ is C(X²)(X²), O, S(O)ₘ or N(X²);
r for each occurrence is independently 1, 2 or 3;
X² for each occurrence is independently hydrogen, optionally substituted (C₁-C₆)alkyl or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halo groups or 1-3 OX³ groups;
X³ for each occurrence is independently hydrogen or (C₁-C₆)alkyl;
X⁶ for each occurrence is independently hydrogen, optionally substituted (C₁-C₆)alkyl, (C₂-C₆)halogenated alkyl, optionally substituted (C₃-C₇)cycloalkyl, (C₃-C₇)-halogenated cycloalkyl, where optionally substituted (C₁-C₆)alkyl and optionally substituted (C₃-C₇)cycloalkyl in the definition of X⁶ is optionally independently mono- or di-substituted with (C₁-C₄)alkyl, hydroxy, (C₁-C₄)alkoxy, carboxyl, CONH₂, -S(O)ₘ(C₁-C₆)alkyl, carboxylate (C₁-C₄)alkyl ester or 1H-tetrazol-5-yl; or when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷ as a ring member;
X⁷ is hydrogen or (C₁-C₆)alkyl optionally substituted with hydroxy;
m for each occurrence is independently 0, 1 or 2;
with the provisos that:
1) X⁶ and X¹² cannot be hydrogen when attached to C(O) or S(O)₂ in the form C(O)X⁶, C(O)X¹², S(O)₂X⁶ or S(O)₂X¹²; and
2) when R⁶ is a bond then L is N(X²) and each r in the definition -(CH₂)ᵣ-L-(CH₂)ᵣ- is independently 2 or 3.

More preferably, the present invention provides such methods and kits
wherein the compound is of the structural formula below, which is designated herein as Formula I-A 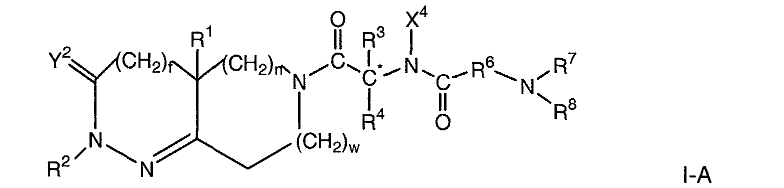 a racemic-diastereomeric mixture or an optical isomer of said compound or a pharmaceutically-acceptable salt or prodrug thereof, or a tautomer thereof,
wherein
f is 0;
n is 0 and w is 2, or n is 1 and w is 1, or n is 2 and w is 0;
Y is oxygen or sulfur;
R¹ is hydrogen, -CN, -(CH₂)_{q}N(X⁶)C(O)X⁶, -(CH₂)_{q}N(X⁶)C(O)(CH₂)ₜ-A¹, -(CH₂)_{q}N(X⁶)SO₂(CH₂)ₜ-A¹, -(CH₂)_{q}N(X⁶)SO₂X⁶, -(CH₂)_{q}N(X⁶)C(O)N(X⁶)(CH₂)ₜ-A¹, -(CH₂)_{q}N(X⁶)C(O)N(X⁶)(X⁶), -(CH₂)_{q}C(O)N(X⁶)(X⁶), -(CH₂)_{q}C(O)N(X⁶)(CH₂)ₜ-A¹, -(CH₂)_{q}C(O)OX⁶, -(CH₂)_{q}C(O)O(CH₂)ₜ-A¹, -(CH₂)_{q}OX⁶, -(CH₂)_{q}OC(O)X⁶, -(CH₂)_{q}OC(O)(CH₂)ₜ-A¹, -(CH₂)_{q}OC(O)N(X⁶)(CH₂)ₜ-A¹, -(CH₂)_{q}OC(O)N(X⁶)(X⁶), -(CH₂)_{q}C(O)X⁶, -(CH₂)_{q}C(O)(CH₂)ₜ-A¹, -(CH₂)_{q}N(X⁶)C(O)OX⁶, -(CH₂)_{q}N(X⁶)SO₂N(X⁶)(X⁶), -(CH₂)_{q}S(O)ₘX⁶, -(CH₂)_{q}S(O)ₘ(CH₂)ₜ-A¹, -(C₁-C₁₀)alkyl, -(CH₂)ₜ-A¹, -(CH₂)_{q}-(C₃-C₇)cycloalkyl, -(CH₂)_{q}-Y¹-(C₁-C₆)alkyl, -(CH₂)_{q}-Y¹-(CH₂)ₜ-A¹ or -(CH₂)_{q}-Y¹-(CH₂)ₜ-(C₃-C₇)cycloalkyl;
where the alkyl and cycloalkyl groups in the definition of R¹ are optionally substituted with (C₁-C₄)alkyl, hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl or 1, 2 or 3 fluoro;
Y¹ is O, S(O)ₘ, -C(O)NX⁶-, -CH=CH-, -C≡C-, -N(X⁶)C(O)-, -C(O)O-, -OC(O)N(X⁶)- or -OC(O)-;
q is 0, 1, 2, 3 or 4;
t is 0, 1, 2 or 3;
said (CH₂)_{q} group and (CH₂)ₜ group may each be optionally substituted with hydroxyl, (C₁-C₄)alkoxy, carboxyl, -CONH₂, -S(O)ₘ(C₁-C₆)alkyl, -CO₂(C₁-C₄)alkyl ester, 1H-tetrazol-5-yl, 1, 2 or 3 fluoro, or 1 or 2 (C₁-C₄)alkyl;
R² is hydrogen, (C₁-C₈)alkyl, -(C₀-C₃)alkyl-(C₃-C₈)cycloalkyl, -(C₁-C₄)alkyl-A¹ or A¹;
where the alkyl groups and the cycloalkyl groups in the definition of R² are optionally substituted with hydroxyl, -C(O)OX⁶, -C(O)N(X⁶)(X⁶), -N(X⁶)(X⁶), -S(O)ₘ(C₁-C₆)alkyl, -C(O)A¹, -C(O)(X⁶), CF₃, CN or 1, 2 or 3 halogen;
R³ is A¹, (C₁-C₁₀)alkyl, -(C₁-C₆)alkyl-A¹, -(C₁-C₆)alkyl-(C₃-C₇)cycloalkyl, -(C₁-C₅)alkyl-X¹-(C₁-C₅)alkyl, -(C₁-C₅)alkyl-X¹-(C₀-C₅)alkyl-A¹ or -(C₁-C₅)alkyl-X¹-(C₁-C₅)alkyl-(C₃-C₇)cycloalkyl;
where the alkyl groups in the definition of R³ are optionally substituted with, -S(O)ₘ(C₁-C₆)alkyl, -C(O)OX³, 1, 2, 3, 4 or 5 halogens, or 1, 2 or 3 OX³; X¹ is O, S(O)ₘ, -N(X²)C(O)-, -C(O)N(X²)-, -OC(O)-, -C(O)O-, -CX²=CX²-, -N(X²)C(O)O-, -OC(O)N(X²)- or -C≡C-;
R⁴ is hydrogen, (C₁-C₆)alkyl or (C₃-C₇)cycloalkyl;
X⁴ is hydrogen or (C₁-C₆)alkyl or X⁴ is taken together with R⁴ and the nitrogen atom to which X⁴ is attached and the carbon atom to which R⁴ is attached and form a five to seven membered ring;
R⁶ is a bond or is 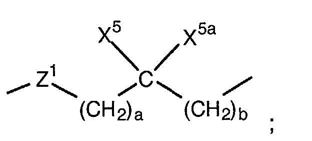 where a and b are independently 0, 1, 2 or 3;
X⁵ and X^{5a} are each independently selected from the group consisting of hydrogen, trifluoromethyl, A¹ and optionally substituted (C₁-C₆)alkyl;
the optionally substituted (C₁-C₆)alkyl in the definition of X⁵ and X^{5a} is optionally substituted with a substituent selected from the group consisting of A¹, OX², -S(O)ₘ(C₁-C₆)alkyl, -C(O)OX², (C₃-C₇)cycloalkyl, -N(X²)(X²) and -C(O)N(X²)(X²);
R⁷ and R⁸ are independently hydrogen or optionally substituted (C₁-C₆)alkyl;
where the optionally substituted (C₁-C₆)alkyl in the definition of R⁷ and R⁸ is optionally independently substituted with A¹, -C(O)O-(C₁-C₆)alkyl, -S(O)ₘ(C₁-C₆)alkyl, 1 to 5 halogens, 1 to 3 hydroxy, 1 to 3 -O-C(O)(C₁-C₁₀)alkyl or 1 to 3 (C₁-C₆)alkoxy; or
R⁷ and R⁸ can be taken together to form -(CH₂)ᵣ-L-(CH₂)ᵣ-;
where L is C(X²)(X²), S(O)ₘ or N(X²);
A¹ in the definition of R¹ is a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen;
A¹ in the definition of R², R³, R⁶, R⁷ and R⁸ is independently (C₅-C₇)cycloalkenyl, phenyl or a partially saturated, fully saturated or fully unsaturated 4- to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6- membered ring, optionally having 1 to 4 heteroatoms independently selected from the group consisting of nitrogen, sulfur and oxygen;
A¹ for each occurrence is independently optionally substituted, in one or optionally both rings if A¹ is a bicyclic ring system, with up to three substituents, each substituent independently selected from the group consisting of F, Cl, Br, I, OCF₃, OCF₂H, CF₃, CH₃, OCH₃, -OX⁶, -C(O)N(X⁶)(X⁶), -C(O)OX⁶, oxo, (C₁-C₆)alkyl, nitro, cyano, benzyl, -S(O)ₘ(C₁-C₆)alkyl, 1 H-tetrazol-5-yl, phenyl, phenoxy, phenylalkyloxy, halophenyl, methylenedioxy, -N(X⁶)(X⁶), -N(X⁶)C(O)(X⁶), -SO₂N(X⁶)(X⁶), -N(X⁶)SO₂-phenyl, -N(X⁶)SO₂X⁶, -CONX¹¹X¹², -SO₂NX¹¹X¹², -NX⁶SO₂X¹², -NX⁶CONX¹¹X¹², -NX⁶SO₂NX¹¹X¹², -NX⁶C(O)X¹², imidazolyl, thiazolyl or tetrazolyl, provided that if A¹ is optionally substituted with methylenedioxy then it can only be substituted with one methylenedioxy;
where X¹¹ is hydrogen or optionally substituted (C₁-C₆)alkyl;
the optionally substituted (C₁-C₆)alkyl defined for X¹¹ is optionally independently substituted with phenyl, phenoxy, (C₁-C₆)alkoxycarbonyl, -S(O)ₘ(C₁-C₆)alkyl 1 to 5 halogens, 1 to 3 hydroxy, 1 to 3 (C₁-C₁₀)alkanoyloxy or 1 to 3 (C₁-C₆)alkoxy;
X¹² is hydrogen, (C₁-C₆)alkyl, phenyl, thiazolyl, imidazolyl, furyl or thienyl, provided that when X¹² is not hydrogen, X¹² is optionally substituted with one to three substituents independently selected from the group consisting of Cl, F, CH₃, OCH₃, OCF₃ and CF₃; or X¹¹ and X¹² are taken together to form -(CH₂)ᵣ-L¹-(CH₂)ᵣ-;
where L¹ is C(X²)(X²), O, S(O)m or N(X²);
r for each occurrence is independently 1, 2 or 3;
X² for each occurrence is independently hydrogen, optionally substituted (C₁-C₆)alkyl, or optionally substituted (C₃-C₇)cycloalkyl, where the optionally substituted (C₁-C₆)alkyl and optionally substituted (C₃-C₇)cycloalkyl in the definition of X² are optionally independently substituted with -S(O)ₘ(C₁-C₆)alkyl, -C(O)OX³, 1 to 5 halogens or 1-3 OX³;
X³ for each occurrence is independently hydrogen or (C₁-C₆)alkyl;
X⁶ is independently hydrogen, optionally substituted (C₁-C₆)alkyl, (C₂-C₆)halogenated alkyl, optionally substituted (C₃-C₇)cycloalkyl, (C₃-C₇)-halogenatedcycloalkyl, where optionally substituted (C₁-C₆)alkyl and optionally substituted (C₃-C₇)cycloalkyl in the definition of X⁶ is optionally independently substituted by 1 or 2 (C₁-C₄)alkyl, hydroxyl, (C₁-C₄)alkoxy, carboxyl, CONH₂, -S(O)ₘ(C₁-C₆)alkyl, carboxylate (C₁-C₄)alkyl ester, or 1 H-tetrazol-5-yl; or
when there are two X⁶ groups on one atom and both X⁶ are independently (C₁-C₆)alkyl, the two (C₁-C₆)alkyl groups may be optionally joined and, together with the atom to which the two X⁶ groups are attached, form a 4- to 9- membered ring optionally having oxygen, sulfur or NX⁷;
X⁷ is hydrogen or (C₁-C₆)alkyl optionally substituted with hydroxyl; and
m for each occurrence is independently 0, 1 or 2;
with the proviso that:
X⁶ and X¹² cannot be hydrogen when it is attached to C(O) or SO₂ in the form C(O)X⁶, C(O)X¹², SO₂X⁶ or SO₂X¹²; and
when R⁶ is a bond then L is N(X²) and each r in the definition -(CH₂)ᵣ-L-(CH₂)ᵣ- is independently 2 or 3.

More preferably, the present invention provides such methods and kits wherein the compound is 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, a prodrug thereof or a pharmaceutically acceptable salt of the compound or the prodrug. Even more preferably, the present invention provides such method wherein the compound is 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide, L-tartrate.

Also, more preferably, the present invention provides such methods and kits wherein the compound is 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide, a prodrug thereof or a pharmaceutically acceptable salt of the compound or the prodrug. Even more preferably, the present invention provides such methods and kits wherein the compound is the (L)-(+)-tartaric acid salt of 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide.

Also, more preferably, the present invention provides such methods and kits wherein the compound is 2-amino-N-(1(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide, a prodrug thereof or a pharmaceutically acceptable salt of the compound or the prodrug. Even more preferably, the present invention provides such method wherein the compound is the (L)-(+)-tartaric acid salt of 2-amino-N-(1(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide.

Even more preferably, the present invention provides such methods and kits wherein the compound is selected from the following: 2-amino-N-(2-(3a-(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl)-isobutyramide, a prodrug thereof or a pharmaceutically acceptable salt of said compound or said prodrug; 2-amino-N-[2-(3a-(R)-benzyl-2-methyl-3-oxo-2,3,3a,4,6,7-hexahydro-pyrazolo[4,3-c]pyridin-5-yl)-1-(R)-benzyloxymethyl-2-oxo-ethyl]-isobutyramide, L-tartrate; 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide, a prodrug thereof or a pharmaceutically acceptable salt of said compound or said prodrug; the (L)-(+)-tartaric acid salt of 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide; 2-amino-N-{1(R)-benzyloxymethyl-2-[1,3-dioxo-8a(S)-pyridin-2-ylmethyl-2-(2,2,2-trifluoro-ethyl)-hexahydro-imidazo[1,5-a]pyrazin-7-yl]-2-oxo-ethyl}-2-methyl-propionamide, a prodrug thereof or a pharmaceutically acceptable salt of said compound or said prodrug; and the (L)-(+)-tartaric acid salt of 2-amino-N-(1(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide.

By the term "growth hormone secretagogue" is meant any exogenously administered compound or agent that directly or indirectly stimulates or increases the endogenous release of growth hormone, growth hormone-releasing hormone or somatostatin in an animal, in particular, a human, or acts as a ghrelin mimetic. The growth hormone secretagogue may be peptidyl or non-peptidyl in nature, however, the use of an orally active growth hormone secretagogue is preferred. In addition, it is preferred that the growth hormone secretagogue induce or amplify a pulsatile release of endogenous growth hormone.

The expression "prodrug" refers to compounds that are drug precursors which, following administration, release the drug *in vivo* via some chemical or physiological process (e.g., a prodrug on being brought to the physiological pH is converted to the desired drug form). A prodrug of a growth hormone secretagogue may be used in the present invention. Exemplary prodrugs are disclosed in the art, particularly in the references cited herein and incorporated herein by reference.

The compounds useful in the present invention may be used alone or in combination with one or more growth hormone secretagogues or with one or more agents which are known to be beneficial for treating bulimia nervosa, male erectile dysfunction, female sexual dysfunction, thyroid cancer, or breast cancer, or ameliorating ischemic nerve or muscle damage. The compounds can be administered in the same dosage form, at the same time or in different dosage forms at the same time or at different times.

Additional examples of growth hormone secretagogues that can be used in the present invention are disclosed in the following International Patent Applications (listed by Publication Nos.), issued U.S. patents and published European patent applications, which are incorporated herein by reference, WO 98/46569, WO 98/51687, WO 98/58947, WO 98/58949, WO 98/58950, WO 99/08697, WO 99/09991, WO 95/13069, U.S. 5,492,916, U.S. 5,494,919, WO 95/14666, WO 94/19367, WO 94/13696, WO 94/11012, U.S. 5,726,319, WO 95/11029, WO 95/17422, WO 95/17423, WO 95/34311, WO 96/02530, WO 96/22996, WO 96/22997, WO 96/24580, WO 96/24587, U.S. 5,559,128, WO 96/32943, WO 96/33189, WO 96/15148, WO 96/38471, WO 96/35713, WO 97/00894, WO 97/07117, WO 97/06803, WO 97/11697, WO 97/15573, WO 97/22367, WO 97/23508, WO 97/22620, WO 97/22004, WO 97/21730, WO 97/24369, U.S. 5,663,171, WO 97/34604, WO 97/36873, WO 97/40071, WO 97/40023, WO 97/41878, WO 97/41879, WO 97/46252, WO 97/44042, WO 97/38709, WO 98/03473, WO 97/43278, U.S. 5,721,251, U.S. 5,721,250, WO 98/10653, U.S. 5,919,777, U.S. 5,830,433 and EP 0995748.

A representative first group of growth hormone secretagogues is set forth in International Patent Application Publication No. WO 97/24369 as compounds having the structural formula below, which is designated herein as Formula II: 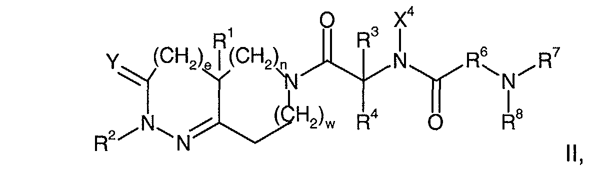 wherein the various substituents are as defined in WO 97/24369. Said compounds are prepared as disclosed therein.

2-Amino-N-(2-(3a-(R)-benzyl-2-methyl-3-oxo-2,3,3a,4,6,7-hexahydropyrazolo-[4,3-c]pyridin-5-yl)-1-(R)-benzyloxymethyl-2-oxo-ethyl)-isobutyramide, having the following structure: 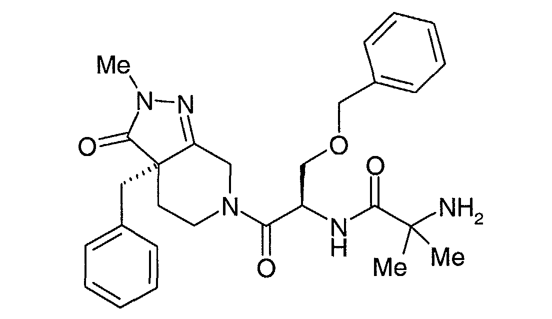 and 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide, having the following structure: 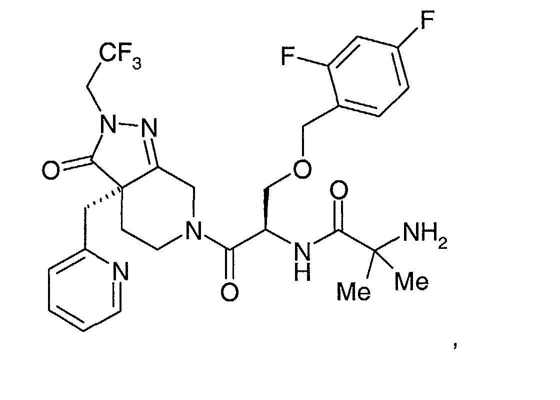 and the pharmaceutically acceptable salts thereof are within the scope of the disclosure of International Patent Application Publication Number WO 97/24369.

A representative second group of growth hormone secretagogues is set forth in International Patent Application Publication No. WO 98/58947, as compounds having the structural formula below, which is designated herein as Formula III: 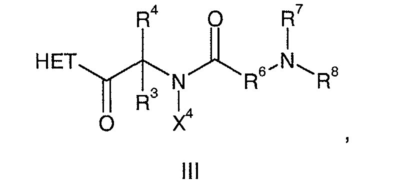 wherein the various substituents are as defined in WO 98/58947. Said compounds are prepared as disclosed therein or as described herein.

The most preferred compound within this second group which may be employed in the present invention is identified as having the following name and structure: 2-amino-N-(1(R)-benzyloxymethyl-2-(1,3-dioxo-8a(S)-pyridin-2-ylmethyl-2-(2,2,2-trifluoro-ethyl)-hexahydro-imidazo[1,5-a]pyrazin-7-yl)-2-oxo-ethyl)-2-methyl-propionamide, 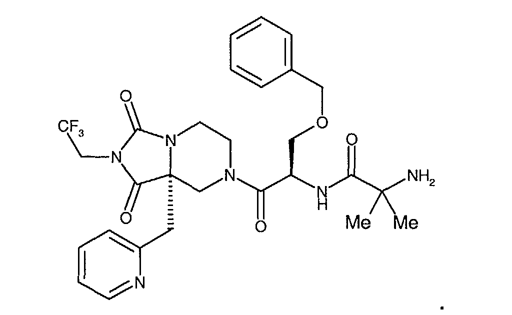 This compound is within the scope of the disclosure of International Patent Application Publication No. WO 98/58947, and may be prepared as described in Examples Five and Six therein.

A representative third group of growth hormone secretagogues is set forth in Published European patent application 0995748, which discloses certain dipeptide growth hormone secretagogues of the structural formula above, which is designated herein as Formula III, and their use for the treatment or prevention of musculoskeletal fraility including osteoporosis.

A representative fourth group of growth hormone secretagogues is set forth in U.S. Patent No. 5,206,235 as having the following structure: 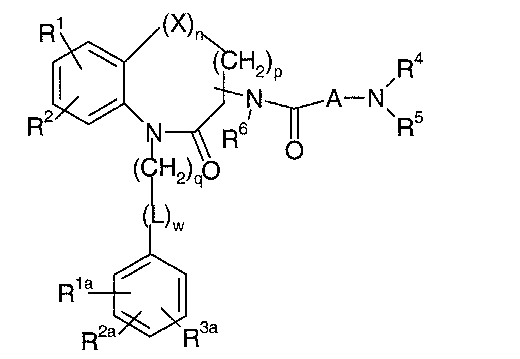 wherein the various substituents are as defined in U.S. Patent No. 5,206,235. Said compounds are prepared as disclosed therein.

The most preferred compounds within this fourth group are identified as having the following structures:

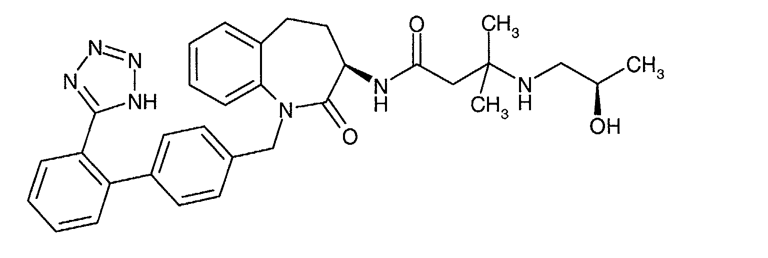

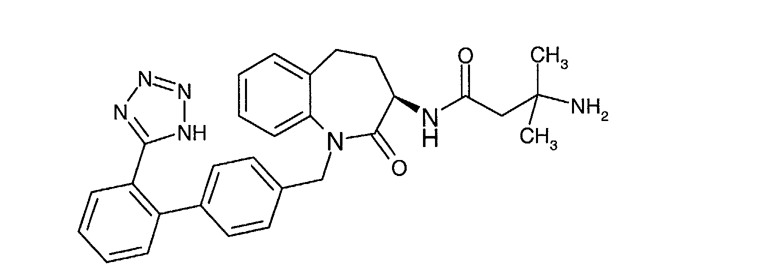

A representative fifth group of growth hormone secretagogues is set forth in U.S. Patent No. 5,283,241, which is incorporated herein by reference, as having the following structural formula: 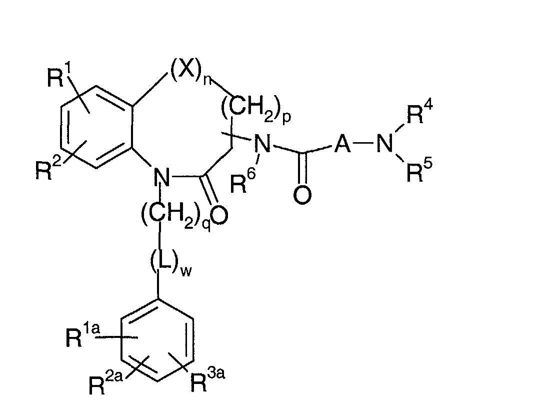 wherein the various substituents are as defined in U.S. Patent 5,283,241. Said compounds are prepared as disclosed therein.

A representative sixth group of growth hormone secretagogues is disclosed in International Patent Application Publication No. WO 97/41879 as compounds having the following structural formulas: 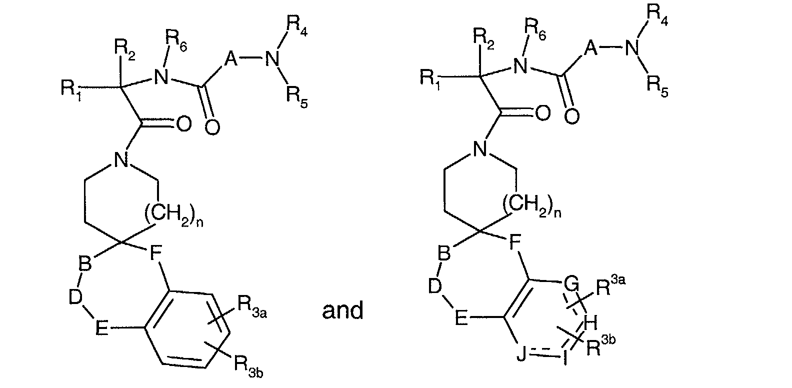 wherein the various substituents are as defined in WO97/41879. Said compounds are prepared as disclosed therein.

The most preferred compounds within this sixth group which may be employed in the present invention are identified as having the following structure: 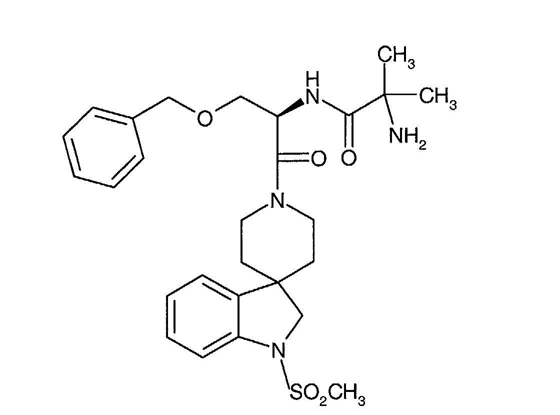 and pharmaceutically acceptable salts thereof, in particular, the methanesulfonate salt.

A representative seventh group of growth hormone secretagogues is disclosed in U.S. Patent No. 5,492,916 as being compounds of the following structural formula: 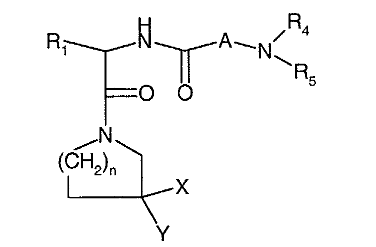 wherein the various substituents are as defined in U.S. Patent No. 5,492,916. Said compounds are prepared as disclosed therein.

The compounds of Formula I used in the methods of the present invention all have at least one asymmetric center as noted, e.g., by the asterisk in the structural Formula I-B below. Additional asymmetric centers may be present in the compounds of Formula I depending upon the nature of the various substituents on the molecule. Each such asymmetric center will produce two optical isomers and it is intended that all such optical isomers, as separated, pure or partially purified optical isomers, racemic mixtures or diastereomeric mixtures thereof, be included within the scope of the methods and kits of the instant invention. In the case of the asymmetric center represented by the asterisk, it has been found that the absolute stereochemistry of the more active and thus more preferred isomer is shown in Formula I-B below: 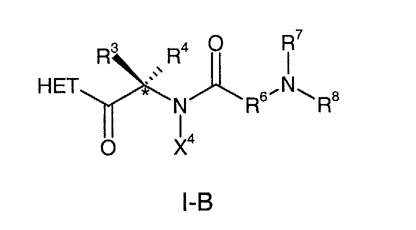 With the R⁴ substituent as hydrogen, the spatial configuration of the asymmetric center corresponds to that in a D-amino acid. In most cases this is also designated an R-configuration although this will vary according to the values of R³ and R⁴ used in making R- or S-stereochemical assignments.

A growth hormone secretagogue can be administered in combination with another compound that can be used to treat bulimia nervosa, male erectile dysfunction, female sexual dysfunction, thyroid cancer, or breast cancer, or ameliorating ischemic nerve or muscle damage.

Examples of other compounds that can be used in combination with a growth hormone secretagogue for the treatment of bulimia nervosa include antidepressants including selective serotonin reuptake inhibitors (SSRIs) such as citalopram (Celexa®), paroxetine (Paxil®), fluoxetine (Prozac®), sertraline hydrochloride (Zoloft®) and fluvoxamine (Luvox®); tricylclic compounds such as amitriptyline (Elvanil®), perphenazine and amitriptyline (Etrafon®), imipramine (Tofranil®), chlordiazepoxide and amitriptyline (Limbitrol®), desipramine (Norpramin®), doxepin (Sinequan®), trimipramine (Surmontil®) and protriptyline (Vivactil®); monoamine oxidase inhibitors such as phenelzine (Nardil®) and tranylcypromine (Parnate®); and other compounds that are used to treat depression such as venlafaxine (Effexor®), mirtazapine (Remeron®), nefazodone (Serzone®) and bupropion (Wellbutrin®).

In addition, a growth hormone secretagogue can be used in combination with estrogen agonists / antagonists, also known as selective estrogen receptor modulators (SERMs), for the treatment of female sexual dysfunction and its component conditions.

Preferred estrogen agonists / antagonists that can be used in combination with a growth hormone secretagogue for the treatment of female sexual dysfunction or its component conditions include the compounds described in U.S. Patent No. 5,552,412. Those compounds are described by formula (IC) given below: 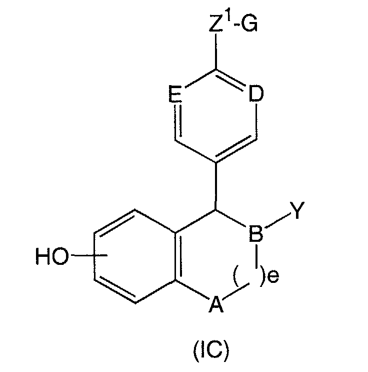 wherein:
A is selected from CH₂ and NR;
B, D and E are independently selected from CH and N;
Y is
   (a) phenyl, optionally substituted with 1-3 substituents independently selected from R⁴;
   (b) naphthyl, optionally substituted with 1-3 substituents independently selected from R⁴;
   (c) C₃-C₈ cycloalkyl, optionally substituted with 1-2 substituents independently selected from R⁴;
   (d) C₃-C₈ cycloalkenyl, optionally substituted with 1-2 substituents independently selected from R⁴;
   (e) a five membered heterocycle containing up to two heteroatoms selected from the group consisting of -O-, -NR²- and -S(O)ₙ-, optionally substituted with 1-3 substituents independently selected from R⁴;
   (f) a six membered heterocycle containing up to two heteroatoms selected from the group consisting of -O-, -NR²- and -S(O)ₙ- optionally substituted with 1-3 substituents independently selected from R⁴; or
   (g) a bicyclic ring system consisting of a five or six membered heterocyclic ring fused to a phenyl ring, said heterocyclic ring containing up to two heteroatoms selected from the group consisting of -O-, -NR²- and -S(O)ₙ-, optionally substituted with 1-3 substituents independently selected from R⁴;
Z¹ is
   (a) -(CH₂)ₚW(CH₂)_{q}-;
   (b) -O(CH₂)ₚCR⁵R⁶-;
   (c) -O(CH₂)ₚW(CH₂)_{q}-;
   (d) -OCHR²CHR³-; or
   (e) -SCHR²CHR³-;
G is
   (a) -NR⁷R⁸;
   (b) 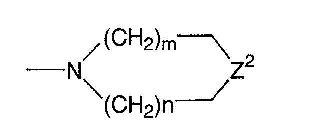 wherein n is 0, 1 or 2; m is 1, 2 or 3; Z² is -NH-, -O-, -S-, or -CH₂-; optionally fused on adjacent carbon atoms with one or two phenyl rings and, optionally independently substituted on carbon with one to three substituents and, optionally, independently on nitrogen with a chemically suitable substituent selected from R⁴; or
   (c) a bicyclic amine containing five to twelve carbon atoms, either bridged or fused and optionally substituted with 1-3 substituents independently selected from R⁴; or
Z¹ and G in combination may be 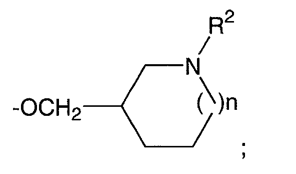
W is
   (a) -CH₂-;
   (b) -CH=CH-;
   (c) -O-;
   (d) -NR²-;
   (e) -S(O)ₙ-;
   (f) 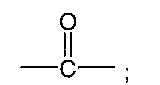
   (g) -CR²(OH)-;
   (h) -CONR²-;
   (i) -NR²CO-;
   (j) 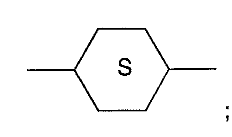 or
   (k) -C≡C-;
R is hydrogen or C₁-C₆ alkyl;
R² and R³ are independently
   (a) hydrogen; or
   (b) C₁-C₄ alkyl;
R⁴ is
   (a) hydrogen;
   (b) halogen;
   (c) C₁-C₆ alkyl;
   (d) C₁-C₄ alkoxy;
   (e) C₁-C₄ acyloxy;
   (f) C₁-C₄ alkylthio;
   (g) C₁-C₄ alkylsulfinyl;
   (h) C₁-C₄ alkylsulfonyl;
   (i) hydroxy (C₁-C₄)alkyl;
   (j) aryl (C₁-C₄)alkyl;
   (k) -CO₂H;
   (l) -CN;
   (m) -CONHOR;
   (n) -SO₂NHR;
   (o) -NH₂;
   (p) C₁-C₄ alkylamino;
   (q) C₁-C₄ dialkylamino;
   (r) -NHSO₂R;
   (s) -NO₂;
   (t) -aryl; or
   (u) -OH;
R⁵ and R⁶ are independently C₁-C₈ alkyl or together form a C₃-C₁₀ carbocyclic ring;
R⁷ and R⁸ are independently
   (a) phenyl;
   (b) a C₃-C₁₀ carbocyclic ring, saturated or unsaturated;
   (c) a C₃-C₁₀ heterocyclic ring containing up to two heteroatoms, selected from -O-, -N- and -S-;
   (d) H;
   (e) C₁-C₆ alkyl; or
   (f) form a 3 to 8 membered nitrogen containing ring with R⁵ or R⁶;
R⁷ and R⁸ in either linear or ring form may optionally be substituted with up to three substituents independently selected from C₁-C₆ alkyl, halogen, alkoxy, hydroxy and carboxy;
a ring formed by R⁷ and R⁸ may be optionally fused to a phenyl ring;
e is 0, 1 or 2;
m is 1, 2 or 3;
n is 0, 1 or 2;
p is 0, 1, 2 or 3;
q is 0, 1, 2 or 3;
and optical and geometric isomers thereof; and nontoxic pharmacologically acceptable acid addition salts, N-oxides, esters, quaternary ammonium salts and prodrugs thereof.

Additional preferred estrogen agonists/antagonists are disclosed in U.S. Patent No.5,552,412 and are described by formula (ID): 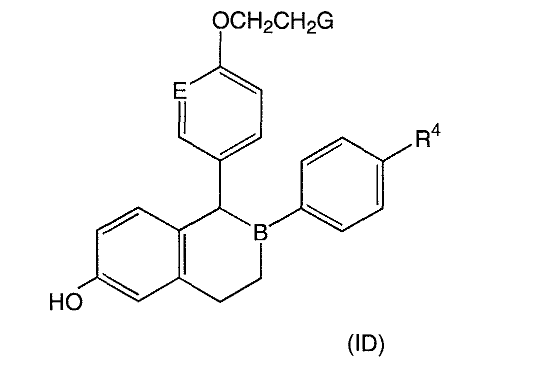 wherein G is

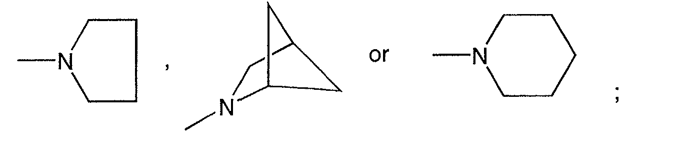

R⁴ is H, OH, F, or Cl; and B and E are independently selected from CH and N.

Especially preferred estrogen agonists/antagonists are:
cis-6-(4-fluoro-phenyl)-5-[4-(2-piperidin-1-yl-ethoxy)-phenyl]-5,6,7,8-tetrahydro-naphthalene-2-ol;
(-)-cis-6-phenyl-5-[4-(2-pyrrolidin-1-yl-ethoxy)-phenyl]-5,6,7,8-tetrahydro-naphthalene-2-ol;
cis-6-phenyl-5-[4-(2-pyrrolidin-1-yl-ethoxy)-phenyl]-5,6,7,8-tetrahydro-naphthalene-2-ol;
cis-1-[6'-pyrrolidinoethoxy-3'-pyridyl]-2-phenyl-6-hydroxy-1,2,3,4-tetrahydronaphthalene;
1-(4'-pyrrolidinoethoxyphenyl)-2-(4"-fluorophenyl)-6-hydroxy-1,2,3,4-tetrahydroisoquinoline;
cis-6-(4-hydroxyphenyl)-5-[4-(2-piperidin-1-yl-ethoxy)-phenyl]-5,6,7,8-tetrahydro-naphthalene-2-ol;

1-(4'-pyrrolidinoethoxyphenyl)-2-phenyl-6-hydroxy-1,2,3,4-tetrahydroisoquinoline and pharmaceutically acceptable salts thereof. An especially preferred salt of (-)-cis-6-phenyl-5-[4-(2-pyrrolidin-1-yl-ethoxy)-phenyl]-5,6,7,8-tetrahydro-naphthalene-2-ol is the tartrate salt.

Other preferred estrogen agonists / antagonists are disclosed in U.S. Patent 5,047,431. The structure of these compounds is given by formula (IV) below: 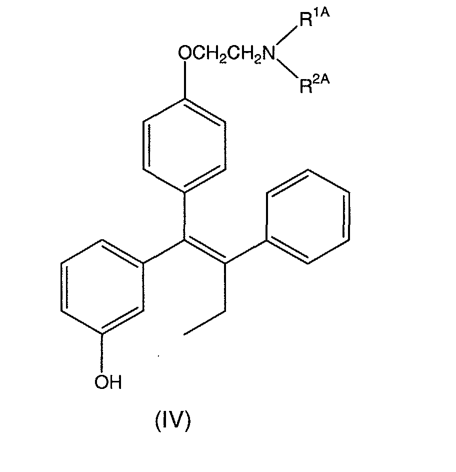 wherein
R^{1A} and R^{2A} may be the same or different and are either H, methyl, ethyl or a benzyl group; and optical or geometric isomers thereof; and pharmaceutically acceptable salts, N-oxides, esters, quaternary ammonium salts, and prodrugs thereof.

Additional preferred estrogen agonists / antagonists are tamoxifen: (ethanamine,2-[-4-(1,2-diphenyl-1-butenyl)phenoxy]-N,N-dimethyl, (Z)-2-, 2-hydroxy-1,2,3-propanetricarboxylate(1:1)) and other compounds as disclosed in U.S. Patent 4,536,516; 4-hydroxy tamoxifen (i.e., tamoxifen wherein the 2-phenyl moiety has a hydroxy group at the 4 position) and other compounds as disclosed in U.S. Patent 4,623,660; raloxifene: (methanone, [6-hydroxy-2-(4-hydroxyphenyl)benzo[b]thien-3-yl][4-[2-(1-piperidinyl)ethoxy]phenyl]-,hydrochloride) and other compounds as disclosed in U.S. Patents 4,418,068; 5,393,763; 5,457,117; 5,478,847 and 5,641,790; toremifene: (ethanamine, 2-[4-(4-chloro-1,2-diphenyl-1-butenyl)phenoxy]-N,N-dimethyl-, (Z)-, 2-hydroxy-1,2,3-propanetricarboxylate (1:1) and other compounds as disclosed in U.S. Patents 4,696,949 and 4,996,225; centchroman: 1-[2-[[4-(-methoxy-2,2, dimethyl-3-phenyl-chroman-4-yl)-phenoxy]-ethyl]-pyrrolidine and other compounds as disclosed in U.S. Patent 3,822,287; idoxifene: pyrrolidine, 1-[-[4-[[1-(4-iodophenyl)-2-phenyl-1-butenyl]phenoxy]ethyl] and other compounds as disclosed in U.S. Patent 4,839,155; 6-(4-hydroxy-phenyl)-5-[4-(2-piperidin-1-yl-ethoxy)-benzyl]-naphthalen -2-ol and other compounds as disclosed in U.S. Patent 5,484,795; and {4-[2-(2-aza-bicyclo[2.2.1]hept-2-yl)-ethoxy]-phenyl}-[6-hydroxy-2-(4-hydroxy-phenyl)-benzo[b]thiophen-3-yl]-methanone and other compounds as disclosed in published international patent application WO 95/10513. Other preferred compounds include GW 5638 and GW 7604, the synthesis of which is described in Willson et al., J. Med. Chem., 1994: 37: 1550-1552.

Further preferred estrogen agonists / antagonists include EM-652 (as shown in formula (V) and EM-800 (as shown in formula (VI)). The synthesis of EM-652 and EM-800 and the activity of various enantiomers is described in Gauthier et al., J. Med. Chem., 1997;40:2117-2122.

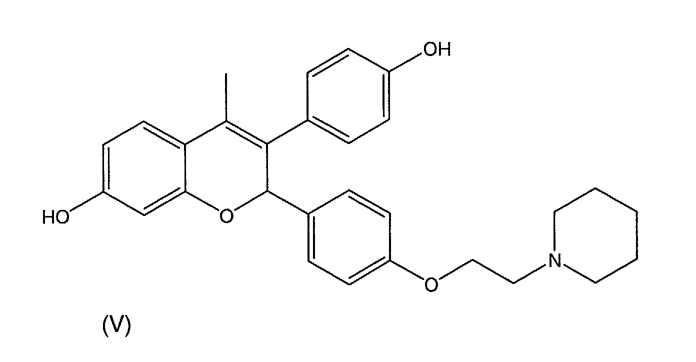

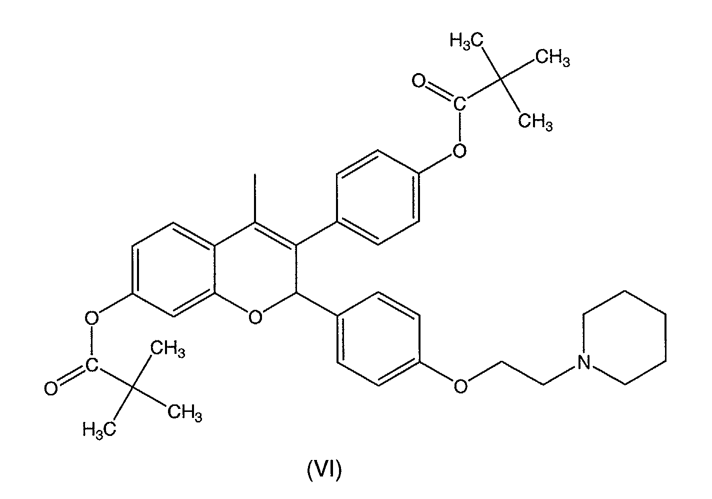

Further preferred estrogen agonists / antagonists include TSE-424 and other compounds disclosed in U.S. Patent 5,998,402, U.S. Patent 5,985,910, U.S. Patent 5,780,497, U.S. Patent 5,880,137, and European Patent Application EP 0802183 A1 including the compounds of the formulas VII and VIII, below: 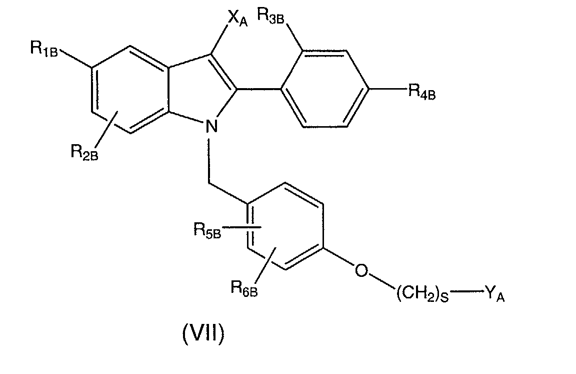 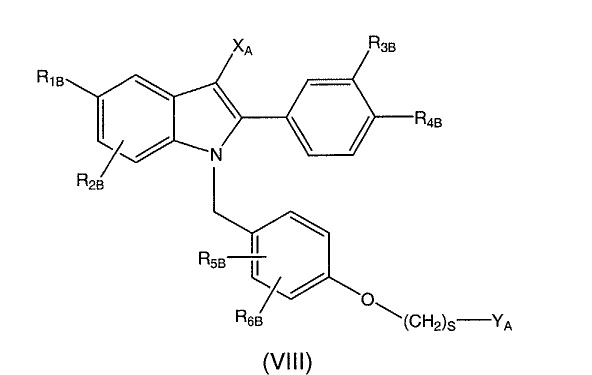 wherein:
R_{1B} is selected from H, OH or the C₁-C₁₂ esters (straight chain or branched) or C₁-C₁₂ (straight chain or branched or cyclic) alkyl ethers thereof, or halogens; or C₁-C₄ halogenated ethers including triflouromethyl ether and trichloromethyl ether.
R_{2B}, R_{3B}, R_{4B}, R_{5B}, and R_{6B} are independently selected from H, OH or the C₁-C₁₂ esters (straight chain or branched) or C₁-C₁₂ alkyl ethers (straight chain or branched or cyclic) thereof, halogens, or C₁-C₄ halogenated ethers including triflouromethyl ether and trichloromethyl ether, cyano, C₁-C₆ alkyl (straight chain or branched), or trifluoromethyl, with the proviso that, when R_{1B} is H, R_{2B} is not OH.
X_{A} is selected from H, C₁-C₆ alkyl, cyano, nitro, triflouromethyl, and halogen; s is 2 or 3;
Y_{A} is selected from:
   a) the moiety: 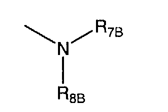 wherein R_{7B} and R_{8B} are independently selected from the group of H, C₁-C₆ alkyl, or phenyl optionally substituted by CN, C₁-C₆ alkyl (straight chain or branched), C₁-C₆ alkoxy (straight chain or branched), halogen, -OH, -CF₃, or -OCF₃;
   b) a five-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   c) a six-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl;
   d) a seven-membered saturated, unsaturated or partially unsaturated heterocycle containing up to two heteroatoms selected from the group consisting of -O-, -NH-, -N(C₁-C₄ alkyl)-, -N=, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, C₁-C₄ alkylamino, di(C₁-C₄)alkylamino, -NHSO₂R_{1B}, -NHCOR_{1B}, -NO₂, and phenyl optionally substituted with 1-3 (C₁-C₄)alkyl; or
   e) a bicyclic heterocycle containing from 6-12 carbon atoms either bridged or fused and containing up to two heteroatoms selected from the group consisting of -O-, -NH-, -N(C₁-C₄ alkyl)-, and -S(O)ᵤ-, wherein u is an integer of from 0-2, optionally substituted with 1-3 substituents independently selected from the group consisting of hydrogen, hydroxyl, halo, C₁-C₄ alkyl, trihalomethyl, C₁-C₄ alkoxy, trihalomethoxy, C₁-C₄ acyloxy, C₁-C₄ alkylthio, C₁-C₄ alkylsulfinyl, C₁-C₄ alkylsulfonyl, hydroxy (C₁-C₄)alkyl, -CO₂H, -CN, -CONHR_{1B}, -NH₂, -N=, C₁-C₄ alkylamino, di(C₁-C₄)alkylamino, -NHSO₂R_{1B}, -NHCOR_{1B}, -NO₂, and phenyl optionally substituted with 1-3 (C₁-C₄) alkyl; and optical or geometric isomers thereof; and nontoxic pharmacologically acceptable acid addition salts, N-oxides, esters, quaternary ammonium salts, and prodrugs thereof.

The more preferred compounds of this invention are those having the general structures V or VI, above, wherein:
R_{1B} is selected from H, OH or the C₁-C₁₂ esters or alkyl ethers thereof, and halogen;
R_{2B}, R_{3B}, R_{4B}, R_{5B}, and R_{6B} are independently selected from H, OH or the C₁-C₁₂ esters or alkyl ethers thereof, halogen, cyano, C₁-C₆ alkyl, or trihalomethyl, preferably trifluoromethyl, with the proviso that, when R_{1B} is H, R_{2B} is not OH;
X_{A} is selected from H, C₁-C₆ alkyl, cyano, nitro, triflouromethyl, and halogen;
Y_{A} is the moiety: 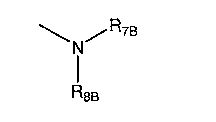
R_{7B} and R_{8B} are selected independently from H, C₁-C₆ alkyl, or combined by -(CH₂)_{w}-, wherein w is an integer of from 2 to 6, so as to form a ring, the ring being optionally substituted by up to three substituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄), -NH₂, C₁-C₄ alkylamino, C₁-C₄ dialkylamino, -NHSO₂(C₁-C₄), -HNCO(C₁-C₄), and -NO₂; and optical and geometric isomers thereof; and nontoxic pharmacologically acceptable acid addition salts, N-oxides, esters, quaternary ammonium salts, and prodrugs thereof.

The rings formed by a concatenated R_{7B} and R_{8B}, mentioned above, may include, but are not limited to, aziridine, azetidine, pyrrolidine, piperidine, hexamethyleneamine or heptamethyleneamine rings.

Preferred compounds of structural formulas VII and VIII, above, are those wherein R_{1B} is OH; R_{2B} - R_{6B} are as defined above; X_{A} is selected from the group of Cl, NO₂, CN, CF₃, or CH₃; Y_{A} is the moiety 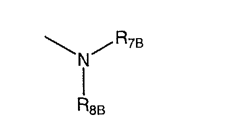 and R_{7B} and R_{8B} are concatenated together as -(CH₂)ₜ-, wherein t is an integer of from 4 to 6, to form a ring optionally substituted by up to three subsituents selected from the group of hydrogen, hydroxyl, halo, C₁-C₄ alkyl, trihalomethyl, C₁-C₄ alkoxy, trihalomethoxy, C₁-C₄ alkylthio, C₁-C₄ alkylsulfinyl, C₁-C₄ alkylsulfonyl, hydroxy (C₁-C₄)alkyl, -CO₂H, -CN, -CONH(C₁-C₄)alkyl, -NH₂, C₁-C₄ alkylamino, di(C₁-C₄)alkylamino, -NHSO₂(C₁-C₄)alkyl, -NHCO(C₁-C₄)alkyl, and -NO₂; and optical and geometric isomers thereof; and nontoxic pharmacologically acceptable acid addition salts, N-oxides, esters, quaternary ammonium salts, and prodrugs thereof including the compound, TSE-424, of formula (VIIa) below:

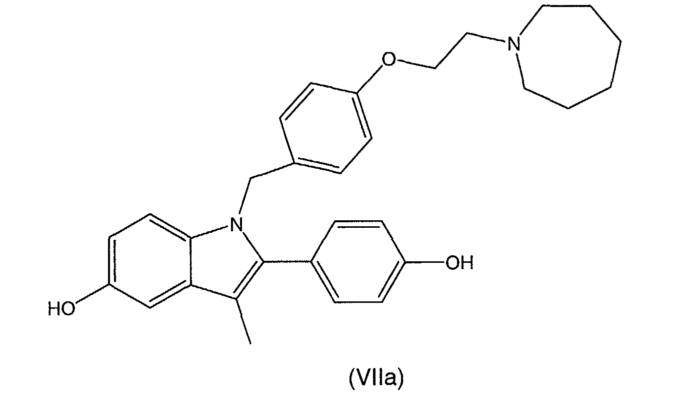

A growth hormone secretagogue can also be used in combination with compounds that can be used to treat erectile dysfunction such as sildenafil citrate (Viagra®) or other PDE5 ligands. Growth hormone secretagogues can also be used in combination with IC351 (Cialis™) or vardenafil. Additional compounds that can be used in combination with growth hormone secretagogues for treating male erectile dysfunction include alprostadil or yohimbine.

Further, for the treatment of female sexual dysfunction and male erectile dysfunction, cGMP elevator agents may be administered in combination with a growth hormone secretagogue.

Preferred as the cGMP elevator are cGMP PDE inhibitors. cGMP PDE inhibitors which are selective for cGMP PDEs rather than cyclic adenosine 3',5'-monophosphate phosphodiesterases (cAMP PDEs) and/or which are selective inhibitors of the cGMP PDEᵥ isoenzyme are particularly preferred. Such particularly preferred cGMP PDE inhibitors are disclosed in US patents 5,250,534; 5,346,901; 5,272,147, and in the international patent application published as WO 94/28902 designating, *inter alia*, the U.S.

Preferred cGMP PDE_{V} (also called PDE5) inhibitors include compounds of formula (IX): 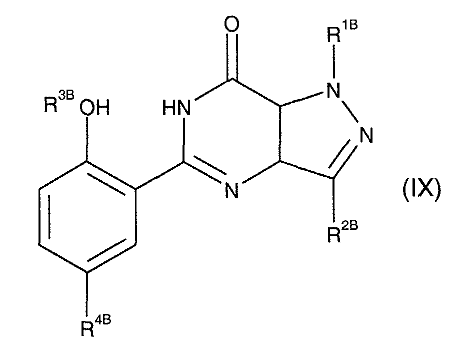 wherein:
R^{1B} is H; C₁-C₃ alkyl; C₁-C₃ perfluoroalkyl; or C₃-C₅ cycloalkyl;
R^{2B} is H; C₁-C₆ alkyl optionally substituted with C₃-C₆ cycloalkyl; C₁-C₃ perfluoroalkyl; or C₃-C₆ cycloalkyl;
R^{3B} is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R^{4B} is C₁-C₄ alkyl optionally substituted with OH, NR^{5B}R^{6B}, CN, CONR^{5B}R^{6B} or CO₂R^{7B}; C₂-C₄ alkenyl optionally substituted with CN, CONR^{5B}R^{6B} or CO₂R^{7B}; C₂-C₄ alkanoyl optionally substituted with NR^{5B}R^{6B}; (hydroxy)C₂-C₄ alkyl optionally substituted with NR^{5B}R^{6B}; (C₂-C₃ alkoxy)C₁-C₂ alkyl optionally substituted with OH or NR^{5B}R^{6B}; CONR^{5B}R⁶B CO₂R^{7B}; halo; NR^{5B}R^{6B}; NHSO₂NR^{5B}R^{6B}; NHSO₂R^{8B}; SO₂NR^{9B}R^{10B} or phenyl, pyridyl, pyrimidinyl, imidazolyl, oxazolyl, thiazolyl, thienyl or triazolyl any of which is optionally substituted with methyl;
R^{5B} and R^{6B} are each independently H or C₁-C₄ alkyl, or together with the nitrogen atom to which they are attached form a pyrrolidinyl, piperidino, morpholino, 4-N(R^{11B})-piperazinyl or imidazolyl group wherein said group is optionally substituted with methyl or OH;
R^{7B} is H or C₁-C₄ alkyl;
R^{8B} is C₁-C₃ alkyl optionally substituted with NR^{5B}R^{6B};
R^{9B} and R^{10B} together with the nitrogen atom to which they are attached form a pyrrolidinyl, piperidino, morpholino or 4-N(R^{12B})-piperazinyl group wherein said group is optionally substituted with C₁-C₄ alkyl, C₁-C₃ alkoxy, NR^{13B}R^{14B} or CONR^{13B}R^{14B};
R^{11B} is H; C₁-C₃ alkyl optionally substituted with phenyl; (hydroxy)C₂-C₃ alkyl; or C₁-C₄ alkanoyl;
R^{12B} is H; C₁-C₆ alkyl; (C₁-C₃ alkoxy)C₂-C₆ alkyl; (hydroxy)C₂-C₆ alkyl; (R^{13B}R^{14B}N)C₂-C₆ alkyl; (R^{13B}R^{14B}NOC)C₁-C₆ alkyl; CONR^{13B}R^{14B}; CSNR^{13B}R^{14B}; or C(NH)NR^{13B}R^{14B}; and
R^{13B} and R^{14B} are each independently H; C₁-C₄ alkyl; (C₁-C₃ alkoxy)C₂-C₄ alkyl; or (hydroxy)C₂-C₄ alkyl;
or a pharmaceutically acceptable salt thereof;
or a pharmaceutically acceptable composition containing either entity.

Preferred cGMP PDE_{V} inhibitors include sildenafil (preferably the citrate salt) {1-[[3-(6,7-dihydro-1-methyl-7-oxo-3-propyl-1 H-pyrazolo[4,3-d]pyrimidin-5-yl)-4-ethoxy-phenyl]sulfonyl]-4-methylpiperazine}, which has the structure of formula (X): 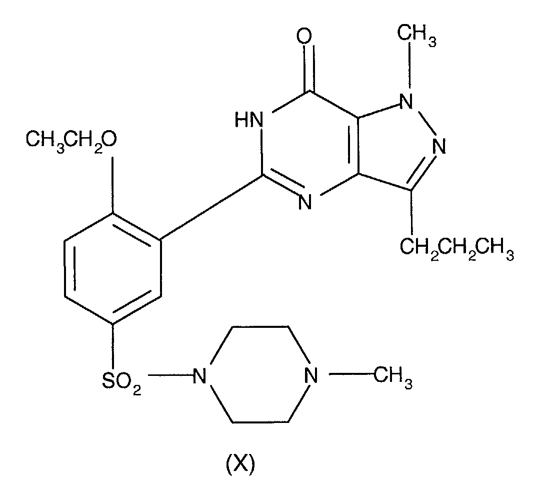 and pharmaceutically acceptable salts thereof, the compound having the structure of formula (XI): 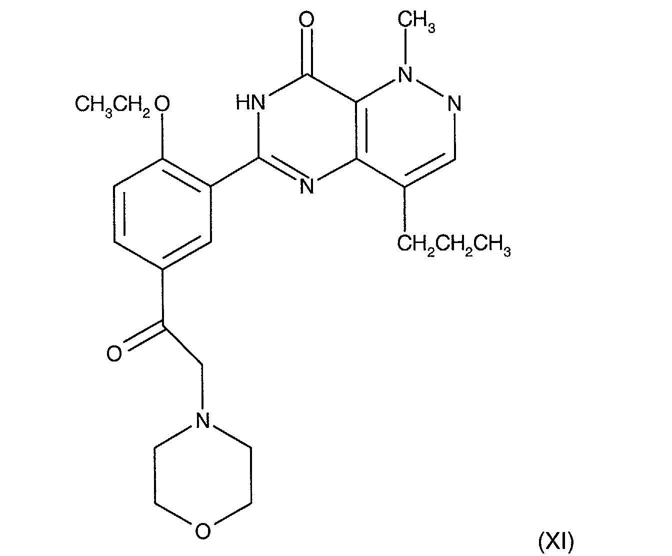 and pharmaceutically acceptable salts thereof, and the compound, 3-ethyl-5-{5-[(4-ethylpiperazino) sulphonyl]-2-(2-methoxyethoxy)pyrid-3-yl}-2-(2-pyridylmethyl)-6,7-dihydro-2H-pyrazolo[4,3-d]pyrimidin-7-one of formula (XII) below:

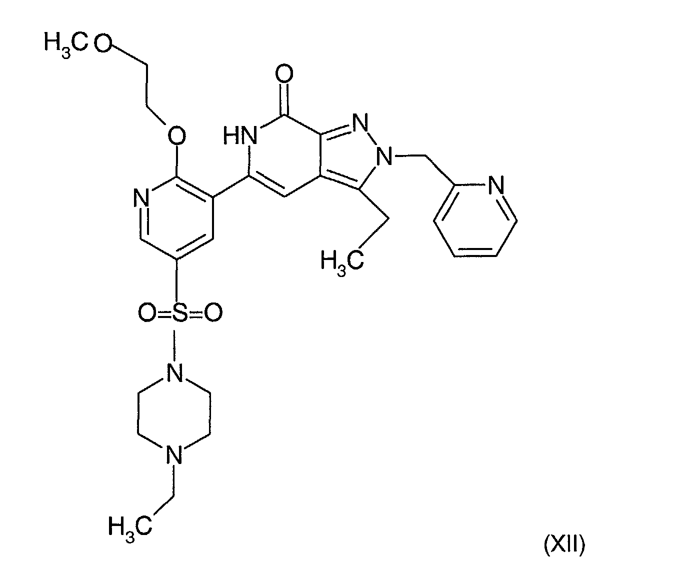

The compound of formula (XI) is disclosed, for example, in US Patents 5,272,147 and 5,426,107.

Also preferred as cGMP PDE_{V} inhibitors are compounds disclosed in PCT/EP95/00183, published as WO 95/19978 and which designates, *inter alia,* the United States, said compounds having the formula (XIII): 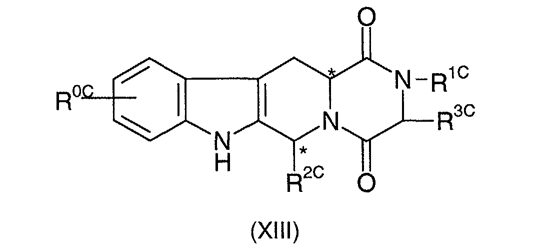 and salts and solvates thereof, in which:
R^{0C} represents hydrogen, halogen or C₁-C₆alkyl,;
R^{1C} represents hydrogen, C₁-C₆alkyl, C₂-C₆alkenyl, C₂-C₆alkynyl, haloC₁-C₆alkyl, C₃-C₈cycloalkyl, C₃-C₈cycloalkylC₁-C₃alkyl, arylC₁-C₃alkyl or heteroarylC₁-C₃alkyl;
R^{2C} represents an optionally substituted monocyclic aromatic ring selected from benzene, thiophene, furan and pyridine or an optionally substituted bicyclic ring 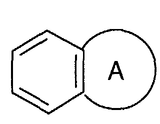 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen; and R^{3C} represents hydrogen or C₁-C₃alkyl, or R^{1C} and R^{3C} together represent a 3- or 4-membered alkyl or alkenyl ring.

A preferred subset of compounds having formula XIIIa (also disclosed in WO 95/19978) includes compounds of the formula: 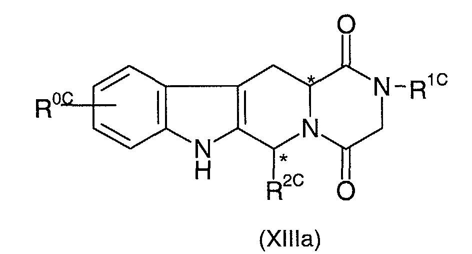 and salts and solvates thereof, in which:
R^{0C} represents hydrogen, halogen or C₁-C₆alkyl;
R^{1C} represents hydrogen, C₁-C₆alkyl, haloC₁-C₆alkyl, C₃-C₈cycloalkyl, C₃-C₈cycloalkyl-C₁-C₃alkyl, arylC₁-C₃alkyl or heteroarylC₁-C₃alkyl; and
R^{2C} represents an optionally substituted monocyclic aromatic ring selected from benzene, thiophene, furan and pyridine or an optionally substituted bicyclic ring 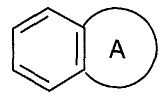 attached to the rest of the molecule via one of the benzene ring carbon atoms and wherein the fused ring A is a 5- or 6-membered ring which may be saturated or partially or fully unsaturated and comprises carbon atoms and optionally one or two heteroatoms selected from oxygen, sulphur and nitrogen.

Suitable cGMP PDE5 inhibitors for the use according to the present invention include: the pyrazolo [4,3-d]pyrimidin-7-ones disclosed in EP-A-0463756; the pyrazolo [4,3-d]pyrimidin-7-ones disclosed in EP-A-0526004; the pyrazolo [4,3-d]pyrimidin-7-ones disclosed in published international patent application WO 93/06104; the isomeric pyrazolo [3,4-d]pyrimidin-4-ones disclosed in published international patent application WO 93/07149; the quinazolin-4-ones disclosed in published international patent application WO 93/12095; the pyrido [3,2-d]pyrimidin-4-ones disclosed in published international patent application WO 94/05661; the purin-6-ones disclosed in published international patent application WO 94/00453; the pyrazolo [4,3-d]pyrimidin-7-ones disclosed in published international patent application WO 98/49166; the pyrazolo [4,3-d]pyrimidin-7-ones disclosed in published international patent application WO 99/54333; the pyrazolo [4,3-d]pyrimidin-4-ones disclosed in EP-A-0995751; the pyrazolo [4,3-d]pyrimidin-7-ones disclosed in published international patent application WO 00/24745; the pyrazolo [4,3-d]pyrimidin-4-ones disclosed in EP-A-0995750; the compounds disclosed in published international application WO95/19978; the compounds disclosed in published international application WO 99/24433 and the compounds disclosed in published international application WO 93/07124.

Preferred type V phosphodiesterase inhibitors for the use according to the present invention include: 5-[2-ethoxy-5-(4-methyl-1-piperazinylsulphonyl)phenyl]-1-methyl-3-n-propyl-1,6-dihydro-7H-pyrazolo[4,3-d]pyrimidin-7-one (sildenafil) also known as 1-[[3-(6,7-dihydro-1-methyl-7-oxo-3-propyl-1H-pyrazolo[4,3-d]pyrimidin-5-yl)-4-ethoxyphenyl]sulphonyl]-4-methylpiperazine (see EP-A-0463756);
5-(2-ethoxy-5-morpholinoacetylphenyl)-1-methyl-3-n-propyl-1,6-dihydro-7H-pyrazolo[4,3-d]pyrimidin-7-one (see EP-A-0526004);
3-ethyl-5-[5-(4-ethylpiperazin-1-ylsulphonyl)-2-n-propoxyphenyl]-2-(pyridin-2-yl)methyl-2,6-dihydro-7H-pyrazolo[4,3-d]pyrimidin-7-one (see WO98/49166);
3-ethyl-5-[5-(4-ethylpiperazin-1-ylsulphonyl)-2-(2-methoxyethoxy)pyridin-3-yl]-2-(pyridin-2-yl)methyl-2,6-dihydro-7H-pyrazolo[4,3-d]pyrimidin-7-one (see WO99/54333);
(+)-3-ethyl-5-[5-(4-ethylplperazin-1-ylsulphonyl)-2-(2-methoxy-1(R)-methylethoxy)pyridin-3-yl]-2-methyl-2,6-dihydro-7H-pyrazolo[4,3-d]pyrimidin-7-one, also known as 3-ethyl-5-{5-[4-ethylpiperazin-1-ylsulphonyl]-2-([(1R)-2-methoxy-1-methylethyl]oxy)pyridin-3-yl}-2-methyl-2,6-dihydro-7H-pyrazolo[4,3-d] pyrimidin-7-one (see WO99/54333);
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see WO01/27113, Example 8);
5-[2-*iso*-Butoxy-5-(4-ethylpiperazin-1-ylsulphonyl)pyridin-3-yl]-3-ethyl-2-(1-methylpiperidin-4-yl)-2,6-dihydro-7H-pyrazolo[4,3-d]pyrimidin-7-one (see WO01/27113, Example 15);
5-[2-ethoxy-5-(4-ethylpiperazin-1-ylsulphonyl)pyridin-3-yl]-3-ethyl-2-phenyl-2,6-dihydro-7H-pyrazolo[4,3-d]pyrimidin-7-one (see WO01/27113, Example 66);
5-(5-acetyl-2-propoxy-3-pyridinyl)-3-ethyl-2-(1-isopropyl-3-azetidinyl)-2,6-dihydro-7*H*-pyrazolo[4,3-*d*]pyrimidin-7-one (see WO01/27113, Example 124);
5-(5-acetyl-2-butoxy-3-pyridinyl)-3-ethyl-2-(1 -ethyl-3-azetidinyl)-2,6-dihydro-7*H*-pyrazolo[4,3-*d*]pyrimidin-7-one (see WO01/27113, Example 132);
(6R,12aR)-2,3,6,7,12,12a-hexahydro-2-methyl-6-(3,4-methylenedioxyphenyl)-pyrazino[2',1':6,1]pyrido[3,4-b]indole-1,4-dione (IC-351), i.e. the compound of examples 78 and 95 of published international application WO95/19978, as well as the compound of examples 1, 3, 7 and 8 therein;
2-[2-ethoxy-5-(4-ethyl-piperazin-1-yl-1-sulphonyl)-phenyl]-5-methyl-7-propyl-3H-imidazo[5,1-f][1,2,4]triazin-4-one (vardenafil) also known as 1-[[3-(3,4-dihydro-5-methyl-4-oxo-7-propylimidazo[5,1-f]-as-triazin-2-yl)-4-ethoxyphenyl]sulphonyl]-4-ethylpiperazine, i.e., the compound of examples 20, 19, 337 and 336 of published international application WO99/24433; and the compound of example 11 of published international application WO93/07124; and compounds 3 and 14 from Rotella, D P, J. Med. Chem., 2000, 43, 1257.

Still other types of cGMP PDE5 inhibitors useful in conjunction with the present invention include:4-bromo-5-(pyridylmethylamino)-6-[3-(4-chlorophenyl)-propoxy]-3(2H)pyridazinone; 1-[4-[(1,3-benzodioxol-5- ylmethyl)amino]-6-chloro-2-quinozolinyl]-4-piperidine-carboxylic acid, monosodium salt; (+)-cis-5,6a,7,9,9,9a-hexahydro-2-[4-(trifluoromethyl)-phenylmethyl-5-methyl-cyclopent-4,5]imidazo[2,1-b]purin-4(3H)one; furazlocillin; cis-2-hexyl-5-methyl-3,4,5,6a,7,8,9,9a-octahydrocyclopent[4,5]-imidazo[2,1-b]purin-4-one; 3-acetyl-1-(2-chlorobenzyl)-2-propylindole-6- carboxylate; 3-acetyl-1-(2-chlorobenzyl)-2-propylindole-6-carboxylate; 4-bromo-5-(3-pyridylmethylamino)-6-(3-(4-chlorophenyl) propoxy)-3-(2H)pyridazinone; I-methyl-5(5-morpholinoacetyl-2-n-propoxyphenyl)-3-n-propyl-1,6-dihydro- 7H-pyrazolo(4,3-d)pyrimidin-7-one; 1-[4-[(1,3-benzodioxol-5-ylmethyl)amino]-6-chloro-2- quinazolinyl]-4-piperidinecarboxylic acid, monosodium salt; Pharmaprojects No. 4516 (Glaxo Wellcome); Pharmaprojects No. 5051 (Bayer); Pharmaprojects No. 5064 (Kyowa Hakko; see WO 96/26940); Pharmaprojects No. 5069 (Schering Plough); GF-196960 (Glaxo Wellcome); E-8010 and E-4010 (Eisai); Bay-38-3045 & 38-9456 (Bayer) and Sch-51866.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice.

Preferably, the cGMP PDE5 inhibitors have an IC₅₀ at less than 100 nanomolar, more preferably, at less than 50 nanomolar, more preferably still at less than 10 nanomolar.

IC₅₀ values for the cGMP PDE5 inhibitors may be determined using established literature methodology, for example as described in EP0463756-B1 and EP0526004-A1.

Preferably the cGMP PDE5 inhibitors used are selective for the PDE5 enzyme. Preferably they are selective over PDE3, more preferably over PDE3 and PDE4. Preferably, the cGMP PDE5 inhibitors have a selectivity ratio greater than 100 more preferably greater than 300, over PDE3 and more preferably over PDE3 and PDE4.

Selectivity ratios may readily be determined by the skilled person. IC₅₀ values for the PDE3 and PDE4 enzyme may be determined using established literature methodology, see S A Ballard *et al.,* Journal of Urology, 1998, vol. 159, pages 2164-2171.

Oral daily dosages of the above cGMP elevators can range from about 1 mg to about 200 mg with a preferred range of from about 20 mg to about 100 mg. Dosage is *ad libitum* from about 15 minutes to about 4 hours prior to sexual activity. Dosages and timing of dosing can be adjusted for topical dosage forms such as creams or aerosols. cGMP elevators of the present invention include produgs, stereoisomers, hydrates, tautomers and salts of the described compounds.

The cGMP PDE inhibitors useful in this invention as cGMP elevators may be chosen from among any of those already known to the art or subsequently discovered and/or hereafter developed. Suitable cGMP PDE inhibitors include those disclosed in any of the following US patents:
a 5-substituted pyrazolo[4,3-d]pyrimidine-7-one as disclosed in US 4,666,908;
a griseolic acid derivative as disclosed in any of US 4,634,706, 4,783,532, 5,498,819, 5,532,369, 5,556,975, and 5,616,600;
a 2-phenylpurinone derivative as disclosed in US 4,885,301;
a phenylpyridone derivative as disclosed in US 5,254,571;
a fused pyrimidine derivative as disclosed in US 5,047,404;
a condensed pyrimidine derivative as disclosed in US 5,075,310;
a pyrimidopyrimidine derivative as disclosed in US 5,162,316;
a purine compound as disclosed in US 5,073,559;
a quinazoline derivative as disclosed in US 5,147,875;
a phenylpyrimidone derivative as disclosed in US 5,118,686;
an imidazoquinoxalinone derivative or its aza analog as disclosed in US 5,055,465 and 5,166,344;
a phenylpyrimidone derivative as disclosed in US 5,290,933;
a 4-aminoquinazoline derivative as disclosed in US 5,436,233 or 5,439,895;
a 4,5-dihydro-4-oxo-pyrrolo[1,2-a]quinoxaline derivative as disclosed in US 5,405,847;
a polycyclic guanine derivative as disclosed in US 5,393,755;
a nitogenous heterocyclic compound as disclosed in US 5,576,322;
a quinazoline derivative as disclosed in US 4,060,615;
a 6-heterocyclyl pyrazolo[3,4-d]pyrimidin-4-one as disclosed in US 5,294,612; and
a 4-aminoquinazoline derivative as disclosed in US 5,436,233;

Other disclosures of cGMP PDE inhibitors include the following, all of which are herein incorporated by reference:
European patent Application (EPA) publication no. 0428268;
European patent 0442204;
International patent application publication no. WO 94/19351;
Japanese patent application 5-222000;
European Journal of Pharmacology, 251, (1994), 1;
International patent application publication no. WO 94/22855;
a pyrazolopyrimidine derivative as disclosed in European patent application 0636626;
a 4-aminopyrimidine derivative as disclosed in European patent application 0640599;
an imidazoquinazoline derivative as disclosed in International patent application WO95/06648;
an anthranilic acid derivative as disclosed in International patent application WO95/18097;
a tetracyclic derivative as disclosed in International patent application WO95/19978;
an imidazoquinazoline derivative as disclosed in European patent application 0668280; and
a quinazoline compound as disclosed in European patent application 0669324.

The cGMP PDE inhibition of a compound can be determined by standard assays known to the art, for example as disclosed in US 5,250,534. Compounds which are selective inhibitors of cGMP PDE relative to cAMP PDE are preferred, and determination of such compounds is also taught in US 5,250,534. Particularly preferred are compounds which selectively inhibit the PDE_{V} isoenzyme, as disclosed in the aforementioned PCT/EP94/01580, published as WO 94/28902.

The growth hormone secretagogues of the present invention may also be used in combination with other compounds that treat cancer, particularly thyroid cancer or breast cancer. Examples of compounds that can be used in combination with a growth hormone secretagogue for the treatment of cancer includes alkylating agents, nitrogen mustards, nitrosoureas, antimetabolites, hormonal agonists /antagonists, all of which are well known to those skilled in the art.

The present invention also provides methods to increase gastrointestinal motility in a patient that has suffered or will suffer from reduced gastrointestinal motility as a result of being administered an agent that decreases gastrointestinal motility. Certain agents are known to cause reduced gastrointestinal motility. Such agents include calcium channel blockers, beta-blockers and narcotics (particularly opiates). The reduction in gastrointestinal motility can be treated by administering to a patient who has or who is going to be administered an agent that causes reduced gastrointestinal motility a therapeutically effective amount of a growth hormone secretagogue, or a pharmaceuticaly acceptable salt or prodrug thereof. The growth hormone secretagogue can be administered to the patient before that patient takes the gastrointestinal motility reducing agent, at the same time or after. Any administration method is contemplated that results in an attenuation or avoidance of reduced gastrointestinal motility. In one embodiment, the growth hormone secretagogue is administered in the same dosage form as the gastrointestinal motility reducing agent. In another embodiment, the growth hormone secretagogue is administered in different dosage forms, at the same time or at different times than the gastrointestinal motility reducing compound.

Growth hormone secretagogues can also be used to increase gastrointestinal motility after surgery. Patients undergoing or having undergone surgery can experience reduced gastrointestinal motility. This reduced gastrointestinal motility can be avoided or ameliorated by administering to a patient who is to undergo or who is undergoing or has undergone surgery a therapeutically effective amount of a growth hormone secretagogue, or a pharmaceutically acceptable salt or prodrug thereof.

Certain compounds used in the present invention may have the potential to exist in different tautomeric forms. All tautomers of a compound of the present invention are within the scope of the present invention. Also, for example, all keto-enol or imine-enamine forms of the compounds are included in the present invention. Those skilled in the art will recognize that the compound names contained herein may be based on a particular tautomer of a compound. While the name for only a particular tautomer may be used, it is intended that all tautomers are encompassed by the name of the particular tautomer and all tautomers are considered part of the present invention.

A compound within the scope of the present invention may exist in unsolvated as well as solvated forms with pharmaceutically acceptable solvents such as water, ethanol, and the like. The present invention contemplates and encompasses both the solvated and unsolvated forms.

Also included within the scope of the present invention are isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in compound and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased *in vivo* half-life or reduced dosage requirements and, hence, may be preferred in some circumstances. Isotopically labelled compounds of this invention and prodrugs thereof can generally be prepared by substituting a readily available isotopically labelled reagent for a non-isotopically labelled reagent.

The expression "pharmaceutically acceptable salts" includes both pharmaceutically acceptable acid addition salts and pharmaceutically acceptable cationic salts. The expression "pharmaceutically-acceptable cationic salts" is intended to include, but is not limited to, such salts as the alkali metal salts, (e.g., sodium and potassium), alkaline earth metal salts (e.g., calcium and magnesium), aluminum salts, ammonium salts, and salts with organic amines such as benzathine (N,N'-dibenzylethylenediamine), choline, diethanolamine, ethylenediamine, meglumine (N-methylglucamine), benethamine (N-benzylphenethylamine), diethylamine, piperazine, tromethamine (2-amino-2-hydroxymethyl-1,3-propanediol) and procaine. The expression "pharmaceutically-acceptable acid addition salts" is intended to include, but is not limited to, such salts as the hydrochloride, hydrobromide, sulfate, hydrogen sulfate, phosphate, hydrogen phosphate, dihydrogenphosphate, acetate, succinate, tartrate, citrate, methanesulfonate (mesylate) and p-toluenesulfonate (tosylate) salts.

The pharmaceutically acceptable acid addition salts of the compounds of this invention may be formed of the compound itself, or of any of its esters, and include the pharmaceutically acceptable salts that are often used in pharmaceutical chemistry. For example, salts may be formed with inorganic or organic acids such as hydrochloric acid, hydrobromic acid, hydroiodic acid, sulfonic acids including such agents as naphthalenesulfonic, methanesulfonic and toluenesulfonic acids, sulfuric acid, nitric acid, phosphoric acid, tartaric acid, pyrosulfuric acid, metaphosphoric acid, succinic acid, formic acid, phthalic acid, lactic acid and the like, most preferably with hydrochloric acid, citric acid, benzoic acid, maleic acid, acetic acid or propionic acid.

The salts of basic compounds can be formed by reacting the compound with a suitable acid. The salts are typically formed in high yields at moderate temperatures, and often are prepared by isolating the compound from a suitable acidic wash as the final step of the synthesis. The salt-forming acid is dissolved in an appropriate organic solvent, or aqueous organic solvent, such as an alkanol, ketone or ester. On the other hand, if a compound is desired in the free base form, it can be isolated from a basic final wash step. A preferred technique for preparing hydrochlorides is to dissolve the free base in a suitable solvent and dry the solution thoroughly, as over molecular sieves, before bubbling hydrogen chloride gas through it. It will also be recognized that it is possible to administer amorphous forms of the compounds.

The term "prodrug" means a compound that is transformed *in vivo* to yield a compound of the present invention. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

For example, if a compound of the present invention contains a carboxylic acid functional group, a prodrug can comprise an ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as α-dimethylaminoethyl), carbamoyl-(C₁-C₂)alkyl, N,N-di(C₁-C₂)alkylcarbamoyl-(C₁-C₂)alkyl or piperidino-, pyrrolidino- or morpholino(C₂-C₃)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl, or α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a compound of the present invention comprises an amine functional group, a prodrug can be formed by the replacement of a hydrogen atom in the amine group with a group such as R^{X}-carbonyl, R^{X}O-carbonyl, NR^{X}R^{X_{'}}-carbonyl where R^{X} and R^{X_{'}} are each independently (C₁-C₁₀)alkyl, (C₃-C₇)cycloalkyl, benzyl, or R^{X}-carbonyl is a natural α-aminoacyl or natural α-aminoacyl-natural α-aminoacyl, -C(OH)C(O)OY^{X} wherein Y^{X} is H, (C₁-C₆)alkyl or benzyl, -C(OY^{X0}) Y^{X1} wherein Y^{X0} is (C₁-C₄) alkyl and Y^{X1} is (C₁-C₆)alkyl, carboxy(C₁-C₆)alkyl, amino(C₁-C₄)alkyl or mono-N- or di-N,N-(C₁-C₆)alkylaminoalkyl, or -C(Y^{X2}) Y^{X3} wherein Y^{X2} is H or methyl and Y^{X3} is mono-N- or di-N,N-(C₁-C₆)alkylamino, morpholino, piperidin-1-yl or pyrrolidin-1-yl.

The identification of a compound as a growth hormone secretagogue, which is able to directly or indirectly stimulate or increase the endogenous release of growth hormone in an animal, may be readily determined by methodology well known in the art, such as the assay described by Smith et al., Science, 260, 1640-1643 (1993) (see text of Figure 2 therein). In a typical experiment, pituitary glands are aseptically removed from 150-200 g Wistar male rats and cultures of pituitary cells are prepared according to Cheng et al., Endocrinol., 124, 2791-2798 (1989). The cells are treated with the subject compound and assayed for growth hormone secreting activity, as described by Cheng et al. (ibid.). In particular, the intrinsic growth hormone secretagogue activity of a compound which may be used in the present invention may be determined by this assay.

The term "patient" means animals, such as humans, companions animals such as dogs, cats and horses, and livestock such as cattle, swine and sheep. Particularly preferred patients are mammals, including both males and females, with humans being even more preferred.

The term "pharmaceutically acceptable" means that a substance or mixture of substances must be compatible with the other ingredients of a formulation, and not deleterious to the patient.

The terms "treating", "treat" or "treatment" include preventive (e.g., prophylactic) and palliative treatment.

The term "therapeutically effective amount" means an amount of a compound that ameliorates, attenuates, or eliminates a particular disease or condition, or prevents or delays the onset of a disease or condition.

In view of their use according to the present invention, the compounds of the present invention may be formulated into various pharmaceutical forms for administration purposes. A compound may be administered, alone or in combination, by oral, parenteral (e.g., intramuscular, intraperitoneal, intravenous or subcutaneous injection, or implant), nasal, vaginal, rectal, sublingual, or topical routes of administration and can be formulated with pharmaceutically acceptable carriers to provide dosage forms appropriate for each route of administration.

Solid dosage forms for oral administration include capsules, tablets, pills, powders and granules and for companion animals the solid dosage forms include an admixture with food and chewable forms. In such solid dosage forms, the compounds and combinations of this invention can be admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings. In the case of chewable forms, the dosage form may comprise flavoring agents and perfuming agents.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as cocoa butter or a suppository wax. Compositions for nasal or sublingual administration are also prepared with standard excipients well known in the art.

The dosage of active ingredients in the compositions, methods and combinations of the present invention invention may be varied; however, it is necessary that the amount of the active ingredients be such that a suitable dosage form is obtained. The selected dosage depends upon the desired therapeutic effect, on the route of administration, and on the duration of the treatment. Generally, dosage levels of between 0.0001 to 100 mg/kg of body weight daily are administered to humans and other animals, e.g., mammals. A preferred dosage range in humans is 0.01 to 5.0 mg/kg of body weight daily which can be administered as a single dose or divided into multiple doses.

A preferred dosage range in animals other than humans is 0.01 to 10.0 mg/kg of body weight daily which can be administered as a single dose or divided into multiple doses. A more preferred dosage range in animals other than humans is 0.1 to 5 mg/kg of body weight daily which can be administered as a single dose or divided into multiple doses.

Where the tartrate salt or other pharmaceutically acceptable salt of the above compounds is used in the present invention, the skilled person will be able to calculate effective dosage amounts by calculating the molecular weight of the salt form and performing simple stoichiometric ratios.

Also, the present invention includes within its scope the use of a compound according to the present invention, alone or in combination with another growth hormone secretagogue, such as those referenced herein, including the growth hormone releasing peptides GHRP-6 and GHRP-1 (described in U.S. Patent No. 4,411,890 and International Patent Applications, Publication Nos. WO 89/07110, WO 89/07111), GHRP-2 (described in WO 93/04081) and B-HT920, as well as hexarelin and growth hormone releasing hormone (GHRH, also designated GRF) and its analogs, growth hormone and its analogs and somatomedins including IGF-I and IGF-II, or in combination with other therapeutic agents, such as α-adrenergic agonists such as clonidine or serotonin 5-HT1D agonists such as sumatriptan, or agents which inhibit somatostatin or its release such as physostigmine and pyridostigmine. Preferably, the compound may be used in combination with growth hormone releasing factor, an analog of growth hormone releasing factor IGF-1 or IGF-II.

Methods to obtain the growth hormone releasing peptides GHRP-6 and GHRP-1 are described in U.S. Patent No. 4,411, 890 and PCT Publications WO 89/07110, WO 89/07111, methods to obtain the growth hormone releasing peptide GHRP-2 are described in PCT Publication WO 93/04081, and methods to obtain hexarelin are described in J. Endocrin. Invest., 15 (Suppl. 4), 45 (1992).

In addition, the present invention includes within its scope the use of a pharmaceutical composition according to the present invention comprising, as an active ingredient, at least one compound of the present invention in association with a pharmaceutical vehicle, carrier or diluent.

The compounds or combination of compounds of the present invention may be formulated into pharmaceutical compositions as known in the art and as discussed herein.

The present invention also relates to kits for the treatment of bulimia nervosa, male erectile dysfunction, female sexual dysfunction, thyroid cancer, breast cancer, or ameliorating ischemic nerve or muscle damage. The present invention also relates to kits useful for increasing gastrointestinal motility after surgery and increasing gastrointestinal motility in patients who have been administered an agent that decreases gastrointestinal motility.

In one embodiment, the kits comprise two separate pharmaceutical compositions: a first pharmaceutical composition comprising a growth hormone secretagogue or a prodrug thereof or a pharmaceutically acceptable salt of said growth hormone secretagogue or said prodrug; and a second therapeutic agent as described herein. The kit comprises a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

In another embodiment of the kits, the kit comprises a pharmaceutical composition comprising a growth hormone secretagogue and instructions for administering the composition for the treatment of bulimia nervosa, male erectile dysfunction, female sexual dysfunction, thyroid cancer, breast cancer, or ameliorating ischemic nerve or muscle damage.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process, recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card e.g., as follows "First Week, Monday, Tuesday, ...etc.... Second Week, Monday, Tuesday,..." etc. Other variations of memory aids will be readily apparent. A "daily dose" can be a single tablet or capsule or several tablets or capsules to be taken on a given day. Also, a daily dose of a second therapeutic agent as describe herein can consist of one tablet or capsule while a daily dose of a compound of the present invention, a prodrug thereof or pharmaceutically acceptable salt of said compound or said prodrug can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

All documents cited herein, including patents and patent applications, are hereby incorporated by reference.

The utility of the compounds described herein in the methods of the present invention are demonstrated by their activity in one or more of the assays described below:

### Assay for Stimulation of Growth Hormone Release from Rat Pituicytes

Compounds having the ability to stimulate GH secretion from cultured rat pituitary cells are identified using the following protocol. This test is also useful for comparison to standards to determine dosage levels.

Cells are isolated from pituitaries of 6-week old male Wistar rats. Following decapitation, the anterior pituitary lobes are removed into cold, sterile Hank's balanced salt solution without calcium or magnesium (HBSS). Tissues are finely minced, then subjected to two cycles of mechanically assisted enzymatic dispersion using 10 U/mL bacterial protease (EC 3.4.24.4, Sigma P-6141, St. Louis, Missouri) in HBSS. The tissue-enzyme mixture is stirred in a spinner flask at 30 rpm in a 5% CO₂ atmosphere at 37 °C for 30 min., with manual trituration after 15 min. and 30 min. using a 10-mL pipet. This mixture is centrifuged at 200 x g for 5 min. Horse serum (35% final concentration) is added to the supernatant to neutralize excess protease. The pellet is resuspended in fresh protease (10 U/mL), stirred for about 30 min. more under the previous conditions, and manually triturated, ultimately through a 23-gauge needle. Again, horse serum (35% final concentration) is added, then the cells from both digests are combined, pelleted (200 x g for about 15 min.), resuspended in culture medium (Dulbecco's Modified Eagle Medium (D-MEM) supplemented with 4.5 g/L glucose, 10% horse serum, 2.5% fetal bovine serum, 1% non-essential amino acids, 100 U/mL nystatin and 50 mg/mL gentamycin sulfate (Gibco, Grand Island, New York) and counted. Cells are plated at 6.0-6.5x10⁴ cells per cm² in 48-well Costar™ (Coastar, Cambridge, Massachusetts) dishes and cultured for 3-4 days in culture medium.

Just prior to carrying out a GH secretion assay, culture wells are rinsed twice with release medium, then equilibrated for 30 minutes in release medium (D-MEM buffered with 25 mM Hepes, pH 7.4 and containing 0.5% bovine serum albumin at 37 °C). Test compositions are dissolved in DMSO, then diluted into pre-warmed release medium. Assays are typically run in quadruplicate. The assay is initiated by adding 0.5 mL of release medium (with vehicle or test compound) to each culture well. Incubation is carried out at 37 °C for 15 minutes, then terminated by removal of the release medium, which is centrifuged at 2000 x g for 15 minutes to remove cellular material. Rat growth hormone concentrations in the supernatants are determined by a standard radioimmunoassay protocol described below.

### Assay for Exogenously-Stimulated Growth Hormone Release in the Rat after Intravenous Administration of Test Compounds

Twenty-one day old female Sprague-Dawley rats (Charles River Laboratory, Wilmington, MA) are allowed to acclimate to local vivarium conditions (24 °C, 12 hr light, 12 hr dark cycle) for approximately 1 week before testing of a compound of this invention. All rats are allowed access to water and a pelleted commercial diet (Agway Country Food, Syracuse NY) *ad libitum.*

On the day of the experiment, test compounds are dissolved in vehicle containing 1% ethanol, 1 mM acetic acid and 0.1% bovine serum albumin in saline. Each test is conducted in three rats. Rats are weighed and anesthetized via intraperitoneal injection of sodium pentobarbital (Nembutol®, 50 mg/kg body weight). Fourteen minutes after anesthetic administration, a blood sample is taken by nicking the tip of the tail and allowing the blood to drip into a microcentrifuge tube (baseline blood sample, approximately 100 µl). Fifteen minutes after anesthetic administration, a test compound is delivered by intravenous injection into the tail vein, with a total injection volume of 1 mL/kg body weight. Additional blood samples are taken from the tail at 5, 10 and 15 minutes after administration of a compound of this invention. Blood samples are kept on ice until serum separation by centrifugation (1430 x g for 10 minutes at 10°C). Serum is stored at -80 °C until serum growth hormone determination by radioimmunoassay as described below.

### Measurement of Rat Growth Hormone

Rat growth hormone concentrations are determined by double antibody radioimmunoassay using a rat growth hormone reference preparation (NIDDK-rGH-RP-2) and rat growth hormone antiserum raised in monkey (NIDDK-anti-rGH-S-5) obtained from Dr. A. Parlow (Harbor-UCLA Medical Center, Torrance, CA). Additional rat growth hormone (1.5U/mg, #G2414, Scripps Labs, San Diego, CA) is iodinated to a specific activity of approximately 30 µCi/µg by the chloramine T method for use as tracer. Immune complexes are obtained by adding goat antiserum to monkey IgG (ICN/Cappel, Aurora, OH) plus polyethylene glycol, MW 10,000-20,000 to a final concentration of 4.3%; recovery is accomplished by centrifugation according to methods well known to those skilled in the art. This assay has a working range of 0.08-2.5 µg rat growth hormone per tube.

### Assessment of Growth Hormone Release in the Dog after Oral Administration

On the day of dosing, the test compound is weighed out for the appropriate dose and dissolved in water. Doses are delivered at a volume of 0.5-3 mL/kg by oral gavage to 2-4 dogs for each dosing regimen. Blood samples (5 mL) are collected from the jugular vein by direct venipuncture pre-dose and at 0.17, 0.33, 0.5, 0.75, 1, 2, 4, 6, 8 and 24 hours post dose using 5 mL vacutainers containing lithium heparin. The prepared plasma is stored at -20 °C until analysis.

### Measurement of Canine Growth Hormone

Canine growth hormone concentrations are determined by a standard radioimmunoassay protocol using canine growth hormone (antigen for iodination and reference preparation AFP-1983B) and canine growth hormone antiserum raised in monkey (AFP-21452578) obtained from Dr. A. Parlow (Harbor-UCLA Medical Center, Torrence, CA). Tracer is produced by chloramine T-iodination of canine growth hormone to a specific activity of 20-40 µCi/µg. Immune complexes are obtained by adding goat antiserum to monkey IgG (ICN/Cappel, Aurora, OH) plus polyethylene glycol, MW 10,000-20,000 to a final concentration of 4.3%; recovery is accomplished by centrifugation according to methods well known to those skilled in the art. This assay has a working range of 0.08-2.5 µg canine GH/tube.

### Assessment of Canine Growth Hormone and Insulin-Like Growth Factor-1 Levels in the Dog after Chronic Oral Administration

The dogs receive test compound daily for either 7 or 14 days. Each day of dosing, the test compound is weighed out for the appropriate dose and dissolved in water. Doses are delivered at a volume of 0.5-3 ml/kg by gavage to 5 dogs for each dosing regimen. Blood samples are collected at days 0, 3, 7, 10 and 14. Blood samples (5 ml) are obtained by direct venipuncture of the jugular vein at pre-dose, 0.17, 0.33, 0.5, 0.75, 1, 2, 3, 6, 8, 12 and 24 hours post administration on days 0, 7 and 14 using 5 ml vacutainers containing lithium heparin for GH determination. In addition, blood is drawn pre-dose and 8 hours after dosing on days 3 and 10 for IGF-I determination. The prepared plasma is stored at -20 °C until analysis.

Plasma samples are extracted with acid ethanol (0.25N HCI in 90% ethanol), centrifuged, then the supernatant is neutralized with tris[hydroxymethyl]amino-methane (TRIZMA base, manufactured by Sigma Chemical Co.) prior to determination of IGF-I concentration using the Nichols Institute IGF-I extraction kit (San Juan Capistrano, CA).

## Claims

1. The use of a compound that is a growth hormone secretagogue or a pharmaceutically acceptable salt or prodrug thereof, in the manufacture of a medicament for treating bulimia nervosa, male erectile dysfunction, female sexual dysfunction, thyroid cancer, or breast cancer, or ameliorating ischemic nerve or muscle damage.

2. The use of a growth hormone secretagogue or a pharmaceutically acceptable salt or prodrug thereof, in the manufacture of a medicament for increasing gastrointestinal motility in a patient who has taken or who is to take an agent that is known to decrease gastrointestinal motility.

3. The use according to claim 2 wherein the agent that is known to decrease gastrointestinal motility is a calcium channel blocker, a beta blocker, or a narcotic.

4. The use of a growth hormone secretagogue or a pharmaceutically acceptable salt or prodrug thereof, in the manufacture of a medicament for increasing gastrointestinal motility after surgery.

5. The use according to any preceding claim wherein the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide or 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide or a pharmaceutically acceptable salt or prodrug thereof.

6. The use according to claim 5 wherein the growth hormone secretagogue is 2-amino-N-[2-(3a(R)-benzyl-2-methyl-3-oxo-2,3,3a,4,6,7-hexahydro-pyrazolo-[4,3-c]pyridin-5-yl)-1-(R)-benzyloxymethyl-2-oxo-ethyl]-isobutyramide, L-tartrate or the L-(+)-tartaric acid salt of 2-amino-N-(1-(R)-(2,4-difluoro-benzyloxymethyl)-2-oxo-2-(3-oxo-3a-(R)-pyridin-2-ylmethyl)-2-(2,2,2-trifluoro-ethyl)-2,3,3a,4,6,7-hexahydro-pyrazolo-[4,3-c]pyridin-5-yl)-ethyl)-2-methyl-propionamide.

7. A kit for the treatment of bulimia nervosa, male erectile dysfunction, female sexual dysfunction, thyroid cancer, or breast cancer, or ameliorating ischemic nerve or muscle damage, the kit comprising:
a. a first pharmaceutical composition that comprises a growth hormone secretagogue, or a pharmaceutically acceptable salt or prodrug thereof; and
b. instructions for administering the pharmaceutical composition to a patient in need thereof to treat bulimia nervosa, male erectile dysfunction, female sexual dysfunction, thyroid cancer, or breast cancer, or ameliorating ischemic nerve or muscle damage.

8. A kit for the treatment of bulimia nervosa, male erectile dysfunction, female sexual dysfunction, thyroid cancer, or breast cancer, or ameliorating ischemic nerve or muscle damage, the kit comprising:
a. a pharmaceutical composition that comprises a growth hormone secretagogue, or a pharmaceutically acceptable salt or prodrug thereof;
b. a second pharmaceutical composition that comprises a second compound that can be used to treat bulimia nervosa, male erectile dysfunction, female sexual dysfunction, thyroid cancer, or breast cancer, or ameliorating ischemic nerve or muscle damage; and
c. instructions for administering the pharmaceutical compositions to a patient in need thereof to treat bulimia nervosa, male erectile dysfunction, female sexual dysfunction, thyroid cancer, or breast cancer, or ameliorating ischemic nerve or muscle damage.
